(19) Europäisches Patentamt
European Patent Office
Office européen des brevets

(11) EP 2 519 511 B1

(12) **EUROPEAN PATENT SPECIFICATION**

(45) Date of publication and mention of the grant of the patent:
**16.12.2015 Bulletin 2015/51**

(21) Application number: **10798147.4**

(22) Date of filing: **29.12.2010**

(51) Int Cl.:
***C07D 317/54*** *(2006.01)* ***C07D 401/12*** *(2006.01)*
***C07D 413/12*** *(2006.01)* ***C07D 295/185*** *(2006.01)*
***C07C 57/42*** *(2006.01)* ***C07C 233/11*** *(2006.01)*
***A61K 31/36*** *(2006.01)* ***A61P 25/22*** *(2006.01)*

(86) International application number:
**PCT/EP2010/070899**

(87) International publication number:
**WO 2011/080313 (07.07.2011 Gazette 2011/27)**

(54) **NOVEL PIPERINE DERIVATIVES AS GABA-A RECEPTORS MODULATORS**
NEUE PIPERINDERIVATE ALS MODULATOREN DER GABA-A REZEPTOREN
NOUVEAUX DÉRIVÉS DE LA PIPÉRINE COMME MODULATEURS DES RÉCEPTEURS GABA-A

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2009 EP 09180957**

(43) Date of publication of application:
**07.11.2012 Bulletin 2012/45**

(73) Proprietor: **Universität Wien**
**1010 Wien (AT)**

(72) Inventors:
- **HERING, Steffen**
  **A-1190 Vienna (AT)**
- **ERKER, Thomas**
  **A-1140 Vienna (AT)**
- **SCHWARZ, Thomas**
  **A-1230 Vienna (AT)**
- **BABURIN, Igor**
  **A-1180 Vienna (AT)**
- **SCHELLMANN, Denise**
  **A-2380 Perchtoldsdorf (AT)**

(74) Representative: **Gassner, Birgitta et al**
**REDL Life Science Patent Attorneys**
**Donau-City-Straße 11**
**1220 Wien (AT)**

(56) References cited:
**EP-A2- 0 002 734**

- **PEDERSEN, MIKAEL E. ET AL: "Amides from Piper capense with CNS activity - a preliminary SAR analysis", MOLECULES , 14(9), 3833-3843 CODEN: MOLEFW; ISSN: 1420-3049 URL: HTTP: //WWW.MDPI.COM/1420-3049/14/9/3833/PD F, 2009, XP002591353, cited in the application**
- **DATABASE CA [Online] CHEMICAL ABSTRACTS SERVICE, COLUMBUS, OHIO, US; DO, GYONG SAM ET AL: "Syntheses and central nervous depressant activities of piperidine derivatives (V). Methylenedioxyphenyl alkenoic acid amides", XP002622906, retrieved from STN Database accession no. 1987:439671 & DO, GYONG SAM ET AL: "Syntheses and central nervous depressant activities of piperidine derivatives (V). Methylenedioxyphenyl alkenoic acid amides", YAKHAK HOECHI , 30(4), 163-8 CODEN: YAHOA3; ISSN: 0513-4234, 1986,**
- **SANGWAN P L ET AL: "Piperine analogs as potent Staphylococcus aureus NorA efflux pump inhibitors", BIOORGANIC & MEDICINAL CHEMISTRY, PERGAMON, GB, vol. 16, no. 22, 15 November 2008 (2008-11-15), pages 9847-9857, XP025608853, ISSN: 0968-0896, DOI: 10.1016/J.BMC.2008.09.042 [retrieved on 2008-09-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 519 511 B1

## Description

### Field of the Invention

This invention relates generally to the field of medicinal chemistry. More specifically, it relates to novel piperine derivatives that have valuable pharmacologic activities, formulations containing such and their use to diagnose, cure or prevent certain diseases.

By changing the structure of piperine new $GABA_A$ receptor modulators have been synthesized with higher efficiency and potency on $GABA_A$ receptors that do not interact with the TRPV1 receptor any longer. The resulting lead compounds have the potential to become drugs for the treatment of anxiety, insomnia, depression, and epilepsy or may be used as general anesthetics.

### Background of the Invention

Epilepsy is one of the most common chronic neurological disorders. The disease is characterized by recurrent seizures, which originate from abnormal and excessive activity of cerebral neurons and result in a paroxysmal disorganization of brain function.

Symptom of the epileptic seizure is in various modes such as disturbance of consciousness, convulsion and automatism depending upon the initial site of sudden cerebral dysrhythmia and way of spreading thereof. In addition, in epilepsy, not only epileptic seizure but also, for example, periodic displeasure, episodic mental disorder, personality change, impairment of intelligence, etc. are often noted.

Insomnia and anxiety are two conditions, which have substantial negative impact on day-to-day quality of life. Insomnia is often described as subjective complaint of poor sleep quality or quantity despite adequate time for sleep. Insomnia results in daytime fatigue, irritability and decreased concentration and is often associated with other diseases such as psychiatric disorders (e.g. anxiety conditions), medical disorders or substance abuse. Anxiety also leads to fatigue, irritability and decreased concentration. Several forms of anxiety are known, such as generalized anxiety disorder, panic disorder, social anxiety disorder and post-traumatic stress disorder. Effective treatment of said conditions thus positively influences day-to-day life of patients suffering from such conditions.

Current pharmaceuticals used for the treatment of insomnia and anxiety comprise benzodiazepines, benzodiazepine-receptor agonists, melatonin-receptor agonists as well as antidepressants. However, most of said drugs exhibit strong adverse effects such as addiction and substance abuse. Naturally-occurring and traditionally used pharmaceuticals comprise herbal substances. However, many patients describe their action as too weak to result in satisfying relief of the conditions outlined above.

On a molecular level, it seems that naturally-occurring substances such as piperine target *inter alia* $GABA_A$ receptors. The pharmacological properties of $GABA_A$ receptors as well as their sensitivity to $\gamma$-aminobutyric acid (GABA) seem to be determined by the subunit composition. The completely assembled receptor is comprised of five subunits. Up to now, 19 isoforms of mammalian $GABA_A$ receptor subunits could be identified: $\alpha$1-6, $\beta$1-3, $\gamma$1-3, $\delta$, $\varepsilon$, $\pi$1-3, $\rho$ and $\sigma$, and it seems that most of the endogenous as well as exogenous ligands bind to interfaces between two of the subunits. $GABA_A$ receptors seem to be implicated in signaling processes involved in diseases such as insomnia and anxiety as already mentioned above, but also processes involved in pain, mood and panic disorders, epilepsy, schizophrenia and symptoms connected to alcohol and/or substance withdrawal/abuse. Therefore, compounds affecting $GABA_A$ receptors may not only be used for treating insomnia and anxiety disorders but possibly also for treating pain, mood and panic disorders, epilepsy, schizophrenia and symptoms connected to alcohol and/or substance withdrawal/abuse.

$GABA_A$ receptors thus represent promising molecular targets for the treatment of *inter alia* insomnia and anxiety.

There is a need for alternative or improved compounds, which act as $GABA_A$ receptor ligands and thus may be used for the treatment of e.g. insomnia and anxiety.

Various natural products modulating $GABA_A$ receptors (e.g. flavonoids, monoterpenes, diterpenes, neolignans, and $\beta$-carbolines) have been identified (Johnston et al., 2006, Tsang et al., 2004). Little is known in most cases, however, about their subunit selectivity, and presently no natural product derived compound is in clinical development.

Piperine is an alkaloid found naturally in plants belonging to the *Piperaceae* family, such as *Piper nigrum,* commonly known as black pepper, and *Piper longum;* commonly known as long pepper. Piperine is the major pungent substance in these plants and is isolated from the fruit of the black pepper and long pepper plants.

Very recently, Pedersen et al. (2009) reported piperine as a $GABA_A$ receptor ligand presumed to bind to the benzodiazepine binding site. However, only weak affinity ($IC_{50}$ of 1.2 mM in a [$^3$H]-flumazenil binding assay was reported and the activity observed by Pedersen et al. might be caused by low-affinity binding to the benzodiazepine binding site at very high piperine concentrations. Furthermore, piperine is, however, known to activate TRPV1 receptors which might cause unwanted side effects such as changes pain and temperature sensitivity (McNamara et al. 2005).

## Summary of the Invention

Accordingly, it is an object of the present invention to provide new compounds which exhibit affinity for the $GABA_A$ receptor and that do not affect TRPV1.

It is another object of certain embodiments of the present invention to provide pharmaceutical compositions comprising new compounds exhibiting affinity for the $GABA_A$ receptor and do not affect TRPV1. In certain embodiments, said new compounds are used for the manufacture of a medicament for treatment of anxiety and/or insomnia. In certain embodiments, said new compounds are used for the manufacture of a medicament for modulating a pharmacological response from the $GABA_A$ receptor.

Pepper is traditionally used in Asian folk medicine as anti-epileptic, anti-anxiety, sedative and sleep inducing herbal preparations (Pei, Y. Q. 1983, Szallasi, A. 2005, and Sunila, E.; Kuttan, G. 2004).

Here, the identification of new piperine derivatives as new positive allosteric modulators of the $GABA_A$ receptors that do not interact with the TRPV1 receptor is described.

The present invention refers to compounds selected from the group consisting of

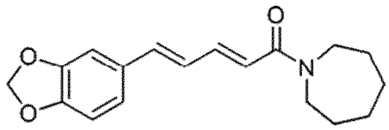,
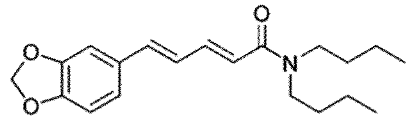,
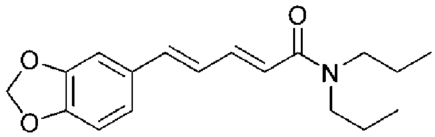, and
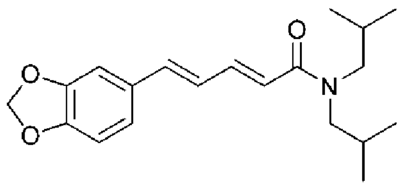

for use as medicament.

A further aspect of the invention is a compound selected from the group consisting of

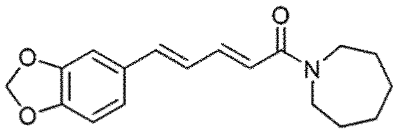,
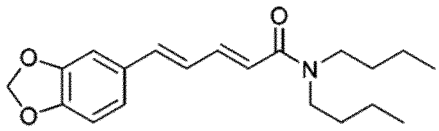,
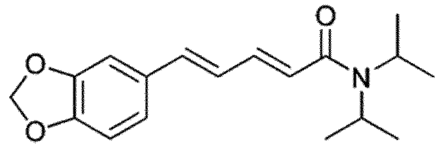,
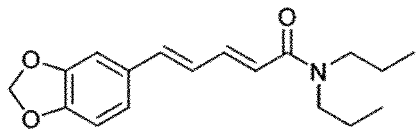,

and

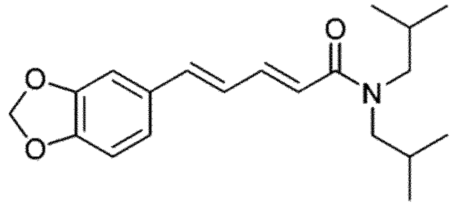

for use in modulating a pharmacological response from the $GABA_A$ receptor.

A further aspect of the invention isa compound as described above, for use in treating or preventing anxiety and/or insomnia, epilepsy, depression, pain, mood and panic disorders, schizophrenia and/or disorders/symptoms connected to alcohol and/or substance withdrawal/abuse.

A further aspect of the invention is a compound as described above, for use in treating or preventing epilepsy or as an anticonvulsant.

A further aspect of the invention is a compound selected from the group consisting of

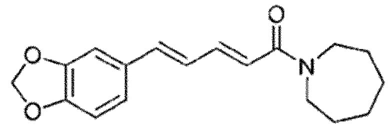
,
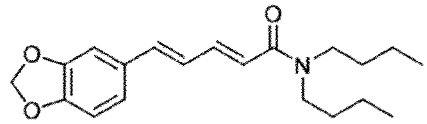

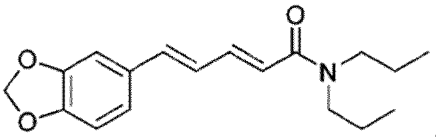
, and
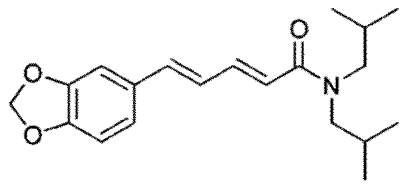

for use in treating or preventing epilepsy or as an anticonvulsant.

A further aspect of the invention is a compound as described above for use in treating or preventing pain.

A further aspect of the invention is a compound selected from the group consisting of

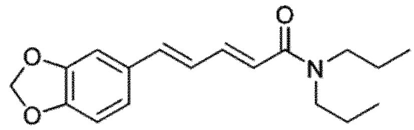
and
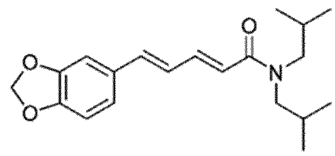

for use in treating or preventing pain.

A further aspect of the invention is a pharmaceutical preparation comprising one or more compounds selected from the group consisting of

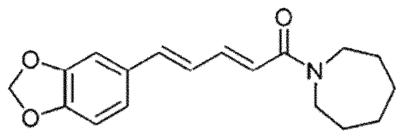
,
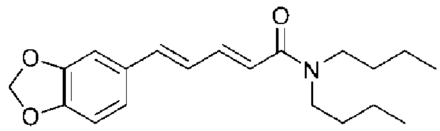

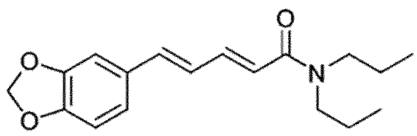
, and
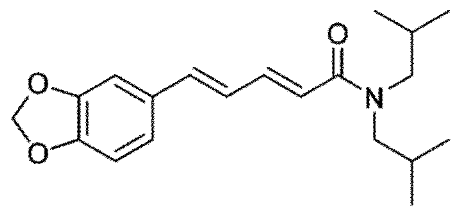

and the pharmaceutically effective salts thereof, in combination with conventional excipients and/or carriers.

A further aspect of the invention is a pharmaceutical preparation comprising one or more compounds selected from the group consisting of

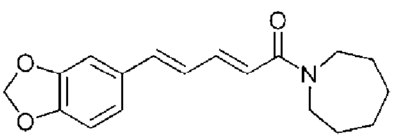
,
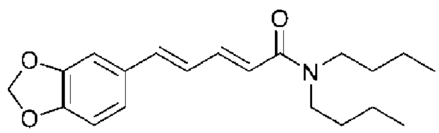
,

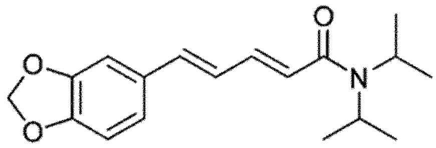,
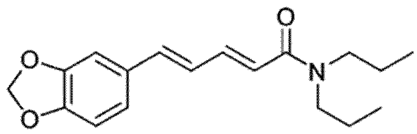

and

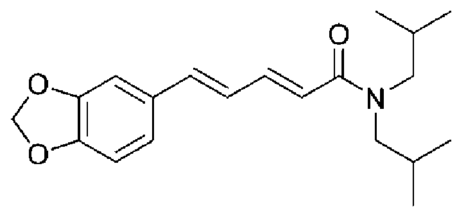

and the pharmacologically effective salts thereof, in combination with conventional excipients and/or carriers for use as an analgesic, anesthetic, sedative, hypnotic, anxiolytic, and/or antiepileptic.

## Detailed Description of the Invention

The benzodiazepines are widely used to treat anxiety, insomnia, epilepsy and depression and other nevouse disease. These drugs may reduce chronic pain and are also used as anesthetics and muscle relaxants. Benzodiazepines can, however, cause undesirable effects including reduced coordination, cognitive impairment, increased accident proneness, physiological dependence, and withdrawal symptoms (Whiting, P. J. 2006, and Rupprecht et al., 2006).

However, piperine is known to activate TRPV1 receptors (McNamara et al. 2005) and is a general inhibitor of both Phase I and Phase II metabolism (Anand et al., 2007) which might cause side effects and drug interactions. Therefore, it was an object of the invention to explore to what extent pharmacological promiscuity of piperamides can be reduced through structural modifications.

## Definitions

As used herein the following definitions apply, unless stated otherwise.

Unless specified otherwise, the term *alkyl,* when used alone or in combination with other groups or atoms, refers to a saturated straight or branched chain consisting of the hydrogen-substituted carbon atoms as indicated, and includes methyl, ethyl, propyl, isopropyl, n-butyl, 1-methylpropyl, isobutyl, t-butyl, 2,2-dimethylbutyl, n-pentyl, 2-methylpentyl, 3-methylpentyl, 4-methylpentyl, n-hexyl and the like.

Unless specified otherwise, the term *alkenyl* refers to a partially unsaturated straight or branched chain consisting of the hydrogen-substituted carbon atoms as indicated that contains at least one double bond, and includes vinyl, allyl, 2-methylprop-1-enyl, but-1-enyl, but-2-enyl, but-3-enyl, buta-1,3-dienyl, penta-1,3-dienyl, penta-2,4-dienyl, 2-methylbut-1-enyl, 2-methylpent-1-enyl, 4-methylpent-1-enyl, 4-methylpent-2-enyl, 2-methylpent-2-enyl, 4-methylpenta-1,3-dienyl, hexen-1-yl and the like.

Unless specified otherwise, the term *alkynyl* refers to a partially unsaturated straight or branched chain consisting of the hydrogen-substituted carbon atoms as indicated that contains at least one triple bond, and includes ethynyl, 1-propynyl, 2-propynyl, 2-methylprop-1-ynyl, 1-butynyl, 2-butynyl, 3-butynyl, 1,3-butadiynyl, 3-methylbut-1-ynyl, 4-methylbut-ynyl, 4-methylbut-2-ynyl, 2-methylbut-1-ynyl, 1-pentynyl, 2-pentynyl, 3-pentynyl, 4-pentynyl, 1 ,3-pentadiynyl, 1,4-pentadiynyl, 3-methylpent-1-ynyl, 4-methylpent-2-ynyl, 4-methylpent-2-ynyl, 1-hexynyl, and the like.

The term heteroalkyl refers to groups which can be derived from alkyl as defined above in its broadest sense by replacing one or more of the groups $-CH_3$ in the hydrocarbon chains independently of one another by the groups -OH, -SH or $-NH_2$, one or more of the groups $-CH_2-$ independently of one another by the groups -O-, -S- or - NH-, one or more of the groups

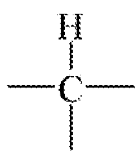

are replaced by the group

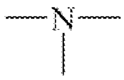

,

one or more of the groups =CH- by the group =N-, one or more of the groups $=CH_2$ by the group =NH or one or more of the groups =CH by the group =N, while in all only a maximum of three heteroatoms may be present in a heteroalkyl, there must be at least one carbon atom between two oxygen and between two sulphur atoms or between one oxygen and one sulphur atom and the group as a whole must have chemical stability.

It follows from the indirect definition/derivation from alkyl that heteroalkyl is made up of the sub-groups of saturated hydrocarbon chains with hetero-atom(s), heteroalkenyl and hetero- alkynyl, while further subdivision into straight-chain (unbranched) and branched may be carried out. If a heteroalkyl is supposed to be substituted, the substitution may take place independently of one another, in each case mono- or polysubstituted, at all the hydrogen-carrying oxygen, sulphur, nitrogen and/or carbon atoms. Heteroalkyl itself may be linked to the molecule as substituent both through a carbon atom and through a heteroatom.

By way of example, the following representative compounds are listed: dimethylamino methyl; dimethylaminoethyl (1-dimethylaminoethyl; 2-dimethylaminoethyl); dimethylaminopropyl (1-dimethylaminopropyl, 2-dimethylaminopropyl, 3-dimethylaminopropyl); diethylamino methyl; diethylaminoethyl (1-diethylamino ethyl, 2-diethylamino ethyl); diethylaminopropyl (1-diethylaminopropyl, 2- diethylamino - propyl, 3 -diethylaminopropyl); diisopropylaminoethyl (1-diisopropylaminoethyl, 2-di-isopropylaminoethyl); bis-2-methoxyethylamino; [2-(dimethylamino-ethyl)-ethyl-amino] -methyl; 3-[2-(dimethylamino-ethyl)-ethyl-amino]-propyl; hydroxymethyl; 2-hydroxy-ethyl; 3-hydroxypropyl; methoxy; ethoxy; propoxy; methoxymethyl; 2-methoxyethyl etc.

Haloalkyl relates to alkyl groups, wherein one or more hydrogen atoms are replaced by halogen atoms. Haloalkyl includes both saturated alkyl groups and unsaturated alkenyl and alkynyl groups, such as for example $-CF_3$, $-CHF_2$, $-CH_2F$, $-CF_2CF_3$, $-CHFCF_3$, $-CH_2CF_3$, $-CF_2CH_3$, $-CHFCH_3$, $-CF_2CF_2CF_3$, $-CF_2CH_2CH_3$, $-CF=CF_2$, $-CCl=CH_2$, $-CBr=CH_2$, $-CI=CH_2$, $-C\equiv C-CF_3$, $-CHFCH_2CH_3$ and $-CHFCH_2CF_3$.

Halogen refers to fluorine, chlorine, bromine and/or iodine atoms.

By cycloalkyl is meant a mono or bicyclic ring, while the ring system may be a saturated ring or, however, an unsaturated, non-aromatic ring, which may optionally also contain double bonds, such as for example cyclopropyl, cyclopropenyl, cyclobutyl, cyclobutenyl, cyclopentyl, cyclopentenyl, cyclohexyl, cyclohexenyl, norbornyl and norbornenyl.

Aryl relates to monocyclic or bicyclic aromatic rings with 6 - 10 carbon atoms such as phenyl and naphthyl, for example.

By heteroaryl are meant mono- or bicyclic aromatic rings, which instead of one or more carbon atoms contain one or more, identical or different hetero atoms, such as e.g. nitrogen, sulphur or oxygen atoms.

Examples include furyl, thienyl, pyrrolyl, oxazolyl, thiazolyl, isoxazolyl, isothiazolyl, pyrazolyl, imidazolyl, triazolyl, tetrazolyl, oxadiazolyl, thiadiazolyl, pyridyl, pyrimidyl, pyridazinyl, pyrazinyl and triazinyl. Examples of bicyclic heteroaryl groups are indolyl, isoindolyl, benzo furyl, benzothienyl, benzoxazolyl, benzothiazolyl, benzisoxazolyl, benzisothiazolyl, benzimidazolyl, indazolyl, isoquinolinyl, quinolinyl, quinoxalinyl, cinnolinyl, phthalazinyl, quinazolinyl and benzotriazinyl, indolizinyl, oxazolopyridyl, imidazopyridyl, naphthyridinyl, indolinyl, isochromanyl, chromanyl, tetrahydroisoquinolinyl, isoindolinyl, isobenzotetrahydrofuryl, isobenzotetrahydro-thienyl, isobenzothienyl, benzoxazolyl, pyridopyridyl, benzotetrahydrofuryl, benzotetrahydrothienyl, purinyl, benzodioxolyl, triazinyl, phenoxazinyl, phenothiazinyl, pteridinyl, benzothiazolyl, imidazopyridyl, imidazothiazolyl, dihydrobenzisoxazinyl, benzisoxazinyl, benzoxazinyl, dihydrobenzisothiazinyl, benzopyranyl, benzothiopyranyl, coumarinyl, isocoumarinyl, chromonyl, chromanonyl, pyridyl-*N*-oxide tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl-*N*-oxide, pyrimidinyl-*N*-oxide, pyridazinyl-*N*-oxide, pyrazinyl-N-oxide, quinolinyl-*N*-oxide, indolyl-*N*-oxide, indolinyl-*N*- oxide, isoquinolyl-*N*-oxide, quinazolinyl-*N*-oxide, quinoxalinyl-*N*-oxide, phthalazinyl-*N*-oxide, imidazolyl-*N*-oxide, isoxazolyl-*N*-oxide, oxazolyl-*N*-oxide, thiazolyl-*N*-oxide, indolizinyl-*N*-oxide, indazolyl-*N*-oxide, benzothiazolyl-*N*-oxide, benzimidazolyl-*N*-oxide, pyrrolyl-*N*-oxide, oxadiazolyl-*N*-oxide, thiadiazolyl-*N*-oxide, triazolyl-*N*-oxide, tetrazolyl-*N*-oxide, benzothiopyranyl-*S*-oxide and benzothiopyranyl-*S,S*-dioxide.

Heterocycloalkyl relates to saturated or unsaturated, non-aromatic mono-, bicyclic or bridged bicyclic rings comprising 3 - 12 carbon atoms, which instead of one or more carbon atoms carry heteroatoms, such as nitrogen, oxygen or sulphur.

Examples of such heterocyloalkyl groups are tetrahydrofuryl, pyrrolidinyl, pyrrolinyl, imidazolidinyl, imidazolinyl, pyrazolidinyl, pyrazolinyl, piperidinyl, piperazinyl, indolinyl, isoindolinyl, morpholinyl, thiomorpholinyl, homomorpholinyl, homopiperidinyl, homopiperazinyl, ho mo thiomorpholinyl, thiomorpholinyl-S-oxide, thiomorpholinyl-*S,S*-dioxide, tetrahydropyranyl, tetrahydrothienyl, homothiomorpholinyl-*S,S*-dioxide, oxazolidinonyl, dihydropyrazolyl, dihydropyrrolyl, dihydropyrazinyl, dihydropyridyl, dihydropyrimidinyl, dihydrofuryl, dihydropyranyl, dioxaborolanyl, tetrahydrothienyl-*S*-oxide, tetrahydrothienyl-S,S-dioxide, homothiomorpholinyl-S-oxide, 2-oxa-5-azabicyclo[2.2.1]heptane, 8-oxa-3-aza-bicyclo[3.2.1]octane, 3,8-diaza-bicyclo[3.2.1]-octane, 2,5-diaza-bicyclo[2.2.1]heptane, 3,8-diaza-bicyclo[3.2.1]octane, 3,9-diaza-bicyclo[4.2.1]nonane and 2.6-diaza-bicyclo[3.2.2]nonane.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The term *pharmaceutically acceptable* means compatible with the treatment of animals, in particular, humans. The term *pharmaceutically acceptable salt* includes both pharmaceutically acceptable acid addition salts and pharmaceutically acceptable basic addition salts.

The term *pharmaceutically acceptable acid addition salt* as used herein means any non-toxic organic or inorganic salt of any base compound of the disclosure, or any of its intermediates. Basic compounds of the disclosure that may form an acid addition salt include, for example, compounds that contain a basic nitrogen atom. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono-, di- or the tri-acid salts can be formed, and such salts may exist in either a hydrated, solvated or substantially anhydrous form. In general, the acid addition salts of the compounds of the disclosure are more soluble in water and various hydrophilic organic solvents, and generally demonstrate higher melting points in comparison to their free base forms. The selection of the appropriate salt will be known to one skilled in the art. Other non-pharmaceutically acceptable acid addition salts, e.g. oxalates, may be used, for example, in the isolation of the compounds of the disclosure, for laboratory use, or for subsequent conversion to a pharmaceutically acceptable acid addition salt.

The term *pharmaceutically acceptable basic salt* as used herein means any non-toxic organic or inorganic basic addition salt of any acid compound of the invention, or any of its intermediates, which are suitable for or compatible with the treatment of animals, in particular humans. Acidic compounds of the invention that may form a basic addition salt include, for example compounds that contain carboxylic acid, sulfonic acid, sulfinic acid, sulfonamide, N-unstubstituted tetrazole, phosphoric acid ester, or sulfuric acid ester. Illustrative inorganic bases which form suitable salts include lithium, sodium, potassium, calcium, magnesium, or barium hydroxide. Illustrative organic bases which form suitable salts include aliphatic, alicyclic or aromatic organic amines such as methylamine, trimethylamine and picoline or ammonia. The selection of the appropriate salt will be known to a person skilled in the art. Other non-pharmaceutically acceptable basic addition salts, may be used, for example, in the isolation of the compounds of the invention, for laboratory use, or for subsequent conversion to a pharmaceutically acceptable acid addition salt. The formation of a desired compound salt is achieved using standard techniques. For example, the neutral compound is treated with a base in a suitable solvent and the formed salt is isolated by filtration, extraction or any other suitable method.

One option to enhance the lipophilicity of a compound is to add an ester group to the parent compound. Due to the enhanced lipophilicity and the higher bioavailability, an enrichment of the compound in the tissue is achieved. After the transfer of the compound to the tissue, the ester group is cleaved off by a naturally produced enzyme (esterase) to expose the active compound.

The term *subject* or *patient* or synonym thereto, as used herein includes all members of the animal kingdom, especially mammals, including human. The subject or patient is suitably a human.

As used herein, and as well understood in the art, *treatment* is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. *Treatment* can also mean prolonging survival as compared to expected survival if not receiving treatment.

*Palliating* a disease or disorder means that the extent and/or undesirable clinical manifestations of a disorder or a disease state are lessened and/or time course of the progression is slowed or lengthened, as compared to not treating the disorder. The term *prevention* or *prophylaxis,* or synonym thereto, as used herein refers to a reduction in the risk or probability of a patient becoming afflicted with the disease or manifesting a symptom associated with the disease.

The term *therapeutically effective amount, effective amount* or *sufficient amount* of a compound of the present invention is a quantity sufficient to, when administered to the subject, including a mammal, for example a human, effect beneficial or desired results, including clinical results, and, as such, an *effective amount* or synonym thereof depends upon the context in which it is being applied.

Moreover, a *treatment* or *prevention regime* of a subject with a therapeutically effective amount of the compound of the present invention may consist of a single administration, or alternatively comprise a series of applications. For example, the compound of the present invention may be administered at least once a week. However, in another embodiment, the compound may be administered to the subject from about one time per week to about three times daily for a given treatment. The length of the treatment period depends on a variety of factors, such as the severity of the disease, the age of the patient, the concentration and the activity of the compounds of the present invention, or a combination thereof. It will also be appreciated that the effective dosage of the compound used for the treatment or prophylaxis may increase or decrease over the course of a particular treatment or prophylaxis regime. Changes in dosage may result and become apparent by standard diagnostic assays known in the art. In some instances, chronic administration may be required.

As used herein the term *administered contemporaneously* means that two substances are administered to a subject in such a way that they are both biologically active in the subject at the same time. The exact details of the administration will depend on the pharmacokinetics of the two substances in the presence of each other, and can include administering one substance within 24 hours of administration of the other, if the pharmacokinetics is suitable. Designs of suitable dosing regimens are routine for one skilled in the art. In particular embodiments, two substances will be administered substantially simultaneously, i.e. within minutes of each other, or in a single composition that comprises both substances.

In understanding the scope of the present disclosure, the term *comprising* and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, *including, having* and their derivatives. Finally, terms of degree such as *substantially, about* and *approximately* as used herein mean a reasonable amount of deviation of the modified term such that the end result is not significantly changed. These terms of degree should be construed as including a deviation of at least $\pm$ 5 % of the modified term if this deviation would not negate the meaning of the word it modifies.

Unless otherwise indicated, the terms a, *an,* and *the* as used herein mean one or more that one.

The present invention also relates to a method of treatment or prevention of a human or non-human mammal suffering from diseases associated with $GABA_A$ receptor modulation in a human or non-human mammal, which comprises administering to said human or non-human mammal in need thereof a therapeutically effective amount of a compound of formula (I) or pharmaceutically acceptable salts, polymorphs, hydrates, tautomers, solvates and stereoisomers thereof, together with pharmaceutically acceptable diluents or carriers. A non-limitative list of such diseases comprises anxiety, epilepsy, sleep disorders, including insomnia, depression, and the like.

As used herein, the term "mammal" shall refer to the Mammalian class of higher vertebrates. The term "mammal" includes, but is not limited to, a human.

If at least one piperine and/or at least one pharmaceutically acceptable salt and/or solvate thereof is/are comprised within a medicament, such a medicament may be formulated for oral, bucal, nasal, rectal, topical or parenteral application. Parenteral application may include intravenous, intramuscular or subcutaneous administration. The at least one piperine derivative and/or at least one pharmaceutically acceptable salt and/or solvate thereof may be applied in pharmaceutically effective amounts, for example in the amounts set out herein below.

Pharmaceutical dosage forms may be solid or liquid dosage forms or may have an intermediate, e.g. gel-like character depending *inter alia* on the route of administration.

In general, the inventive solid dosage forms will comprise various pharmaceutically acceptable excipients which will be selected depending on which functionality is to be achieved for the dosage form.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants.

Typical pharmaceutically acceptable excipients include substances like sucrose, manitol, sorbitol, starch and starch derivatives, lactose, and lubricating agents such as magnesium stearate, disintegrants and buffering agents.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents. When administered by nasal aerosol or inhalation, the compositions according to the present invention may be prepared as solutions in a saline, employing benzyl alcohol or other suitable preservatives, absorption promoters to enhance bioavailability fluorocarbons and/or other solubilising or dispersing agents.

Further conventional excipients, which can be used in the aforementioned dosage forms depending on the functionality that is to be achieved for the dosage form, include pharmaceutically acceptable organic or inorganic carrier substances which do not react with the active compound. Suitable pharmaceutically acceptable carriers include, for

instance, water, salt solutions, alcohols, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants.

The person skilled in the art is aware that bioavailability of the at least one piperine derivative and/or its pharmaceutically acceptable salt and/or solvate can be enhanced by micronisation of the formulations and the actives using conventional techniques such as grinding, milling and spray-drying in the presence of suitable excipients or agents such as phospholipids or surfactants.

Injectable preparations of at least one piperine derivative and/or pharmaceutically acceptable salt and/or solvent thereof, for example sterile injectable aqueous or oleaginous suspensions, can be formulated according to the known art using suitable dispersing agents, wetting agents and/or suspending agents. A sterile injectable preparation can also be a sterile injectable solution or suspensionin a non-toxic parenterally acceptable diluant or solvent. Among the acceptable vehicles and solvents that can be used are water and isotonic sodium chloride solution. Sterile oils are also conventionally used as solvent or suspending medium.

Suppositories for rectal administration of at least one piperine derivative and/or pharmaceutically acceptable salt and/or solvent thereof can be prepared by e.g. mixing the compounds or compositions with a suitable non-irritating excipient such as cocoa butter and polyethylene glycols which are solid at room temperature but liquid at rectal temperature such that they will melt in the rectum and release the at least one piperine derivative and/or pharmaceutically acceptable salt and/or solvent thereof from said suppositories.

Oral dosage forms may be a particularly preferred embodiment in view of patients' overall acceptance of this type of dosage forms. Oral dosage forms may be liquid or solid. Solid oral dosage forms can include e.g. tablets, troches, pills, capsules, powders, effervescent formulations, dragees and granules.

According to another aspect of the present invention, the solid dosage form comprises a film coating. For example, the inventive dosage form may be in the form of a so-called film tablet. According to some aspects, the inventive dosage may comprise two or more film coating layers. The corresponding dosage form may be a bilayer or multilayer tablet.

As outlined above, suitable dosage forms according the present invention may be in the form of a tablet, a dragee or a capsule. The capsule may be a two-piece hard gelatin capsule, a two-piece hydroxypropylmethylcellulose capsule, a two-piece capsule made of vegetable or plant-based cellulose or a two-piece capsule made of polysaccharide. The tablet may be a compressed tablet and/or a film coated tablet.

In one embodiment, the oral dosage forms may be formulated to ensure an immediate release of the at least one piperine derivative and/or its pharmaceutically acceptable salt and/or solvate.

In another embodiment, the oral dosage forms may be formulated to ensure a controlled release of the at least one piperine derivative and/or its pharmaceutically acceptable salt and/or solvate. Such dosage forms may therefore be designated as controlled release (CR) pharmaceutical dosage forms.

The term "controlled release dosage form" in the context of the present invention is used to highlight that a pharmaceutical dosage form is not an immediate release (IR) pharmaceutical dosage form. An oral immediate release pharmaceutical dosage form will typically release substantially all of the at least one piperine and/or its pharmaceutically acceptable salt and/or solvate within a short time after administration. Typically, an IR dosage form will have released 70 % by weight of the pharmaceutically active agents within thirty minutes of administration. The release rates may be determined using the European Pharmacopoeia Paddle Method.

A controlled release dosage form may designate a pharmaceutical dosage form that releases the active agent only after the dosage form has reached a certain site of the body, i.e. the stomach or the gastro-intestinal tract. Additionally or alternatively it may designate a dosage form, which releases the active agent over a prolonged period of time. In the latter case, a controlled release dosage form may be designated as a sustained release dosage form.

A site-specific controlled release of the pharmaceutically active agent, being in the present case at least one piperine derivative and/or a pharmaceutically acceptable salt and/or solvate thereof, may e.g. be achieved in that the release is made dependent on the pH value of the liquids that the dosage form encounters when passing through the human body. Such a pH-dependent release may allow that a dosage form releases the active agent not in the stomach, but only in the gastro-intestinal tract. Another embodiment would be that such a controlled release dosage form releases the active agent once it enters the body. Typical examples of controlled release dosage form which pH-independently release the active agent are dosage forms that comprise an enteric coating.

The term "sustained release" instead refers to the release of the pharmaceutically active compounds from the dosage form over an extended period of time but not necessarily to the release at a defined place. In general, sustained release in the context of the present invention means that a pharmaceutically active agent such as at least one piperine

derivative and/or its pharmaceutically acceptable salt and/or solvate are released from the pharmaceutical dosage form over a time period of at least 2 hours. Of course, the release of the pharmaceutically active agent from the dosage form may also take place over time periods of at least 4 hours, at least 6 hours, at least 10 hours, at least 12 hours or at least 14 hours.

The sustained release characteristics of a dosage form may be adapted such that a therapeutic effect is achieved for at least 8 hours, for at least 12 hours or for at least 24 hours. Such pharmaceutical dosage forms have the advantage that they can be administered on a 3-times, 2-times or once-a-day basis to the patient.

Of course, the above principles can be combined. For example, a pharmaceutical dosage form may comprise an enteric coating in order to ensure that the active agent is released only in the gastro-intestinal tract. The release during the gastro-intestinal passage may, however, display the characteristics of sustained release.

Additionally and/or alternatively the principles of immediate release and sustained release may be combined. Thus, a dosage form may comprise an immediate release phase that ensures a quick onset of therapeutic action that is then prolonged by a second phase of the pharmaceutical dosage form ensuring sustained release characteristics.

Sustained release characteristics can be achieved by different formulation approaches. For example, a pharmaceutical dosage form may comprise a sustained release matrix in which the pharmaceutically active agent such as the at least one piperine derivative and/or its pharmaceutically acceptable salt and/or solvate is embedded in order to achieve the sustained release properties of the dosage form.

In another embodiment, a sustained release coating may be used to ensure the sustained release characteristics of the dosage form. In such a case, the pharmaceutically active agent such as the at least one piperine derivative and/or its pharmaceutically acceptable salt and/or solvate may be applied on/or within e.g. a carrier, which has no substantial influence on the release of the active agent. This drug-loaded carrier may then be overcoated with a corresponding sustained release coating.

These approaches for achieving sustained release of a pharmaceutically active agent, i.e. use of a matrix or a coating may of course, also be combined. The person skilled in the art is further aware of other technical approaches for achieving a sustained release of the dosage form which include e.g. osmotically driven sustained dosage forms.

Typically, if a sustained release matrix system is used, the pharmaceutically active agent such as the at least one piperine derivative and/or its pharmaceutically acceptable salt and/or solvate will be dispersed throughout a matrix-forming material. The matrix-forming materials may be chosen to achieve an erosive matrix, a diffusion matrix or a matrix system, which combines the characteristics of an erosive and a diffusion matrix. Suitable materials for inclusion in a sustained release matrix include hydrophilic or hydrophobic polymers including cellulose ether and preferably alkyl celluloses and hydroxyl alkyl celluloses as well as acrylic resins. Other materials that may be used in a sustained release matrix may be fatty alcohols, fatty acids or polyethylene glycols. The person skilled in the art will be aware of how to build such pharmaceutical dosage forms.

According to another aspect of the present invention, the dosage form contains the at least one piperine derivative and/or its pharmaceutically acceptable salt and/or solvate compressed together with an excipient. It may be especially preferred to prepare the inventive dosage from the at least one piperine derivative and/or its pharmaceutically acceptable salt and/or solvate with a polymer. In this context, suitable polymers according to the present invention may be selected form the group comprising alginic acid, carboxymethylcellulose calcium, carboxymethylcellulose sodium, colloidal silicon dioxide, guar gum, magnesium aluminum silicate, methylcellulose, microcrystalline cellulose, cellulose, pregelatinized starch, sodium alginate, starch, ethylcellulose, gelatin, hydroxyethyl cellulose, hydroxypropyl cellulose, hydroxypropylmethyl cellulose, polymethacrylate, povidone, shellac and zein.

The pharmaceutical compositions in accordance with the present invention may not only comprise the at least one piperine derivative and/or its pharmaceutically acceptable salt and/or solvate but also another pharmaceutically active agents and preferably one or more pharmaceutically active agents which are known to have a positive effect on the treatment and/or prevention of e.g. insomnia and anxiety when administered to a patient in need of treatment thereof.

As regards human patients, the at least one piperine derivative and/or pharmaceutically acceptable salt and/or solvate thereof may be administered to a patient in an amount of about 1 μg to 5.000 mg, preferably of about 0.01 to about 1.000 mg/kg body weight per day, more preferably in an amount of about 0.1 mg/kg to about 100 mg/kg body weight per day. A human patient may in particular be treated with about 0.05 mg to about 1.500 mg and more specifically with about 0.3 mg per day of the at least one piperine derivative and/or pharmaceutically acceptable salt and/or solvent thereof.

Another suitable criterion for selecting an appropriate amount of the at least one piperine derivative and/or of a pharmaceutically acceptable salt and/or solvate thereof is that the at least one piperine derivative and/or pharmaceutically acceptable salt and/or solvate thereof may be administered to an individual in an amount of about 0.01 to about 100 mg/kg/d, preferably in an amount ofabout 0.05 to about 50 mg/kg/d, more preferably in an amount of about 0.1 to about 25 mg/kg/d and in particular in an amount of about 0.5 to about 15 mg/kg/d.

Particularly preferred is the formulation of a medicament for the oral application. In an embodiment where at least one piperine derivative is used for the oral application, one will consider to use about 0.01 to about 10.000 mg,

about 0.25 to about 5.000 mg, about 0.5 to about 1.500 mg or about 0.25 to about 500 mg of the at least one piperine derivative or at least one pharmaceutically acceptable salt and/or solvate thereof for the pharmaceutical compositions, uses and methods for treating inter alia insomnia and anxiety as mentioned above.

These amounts can be administered at once or as multiple doses (at least 2, 3, 4, 5 or 10 doses) per day.

A pharmaceutical composition comprising at least one compound of the present invention may be used for the treatment of a disorder selected from the group of disorders comprising insomnia, anxiety, pain, mood and panic disorders, epilepsy, schizophrenia and disorders/symptoms connected to alcohol and/or substance withdrawal/abuse. A pharmaceutical composition comprising at least one compound of the present invention may thus *inter alia* be used as analgesic, anesthetic, sedative, hypnotic, anxiolytic, and/or antiepileptic.

**Brief description of the drawings:**

Figure 1:
Potentiation of $I_{GABA}$ through piperine and piperine derivatives. Each data point represents means ± S.E.M. from at least 2-7 oocytes from ≥ 2 batches.

Figure 2:
Concentration response curves for SCT-29, SCT-64, SCT-66 compared with piperine (dotted line).

Figure 3:
Activation of cation current trough TRPV1 receptors by piperine (100 μM) and its derivatives (100 μM). Current induced by piperine was defined as 100%.

Figure 4:
Modulation of TRPV1 by piperine and its derivatives in oocytes.
Figure 4 shows a cation current through TRPV1 receptors induced by application of 300 μM piperine while no current was induced upon application of 300 μM SCT-29, SCT-46, SCT-64, SCT-66, and SCT-68 to the oocytes. This indicates that piperine based derivatives, which modulate $GABA_A$ receptors (Fig. 2) do not activate TRPV1 receptors. In other words, compounds SCT-29, SCT-46, SCT-64, SCT-66, and SCT-68 do not activate TRPV1 compared to piperine, even in the applied high concentration (300 μM, see Fig. 4).

Figure 5:
Behavior in the Elevated Plus Maze Test for control and piperine-treated mice at the indicated doses. Bars indicate **(A)** the time spent on the open arms (OA) in % of the total time and in **(B)** the total distance travelled, respectively. Bars represent means ± S.E.M. from at least 8 different mice (*) indicates statistically significant (p<0.05) differences.

Figure 6:
Behaviour in the Open Field Test for control and piperine-treated mice at the indicated doses. Bars indicate the total ambulation. Bars represent means ± S.E.M. from at least 8 mice (*) indicates statistically significant (p<0.05) differences.

Figure 7:
Effect of piperine on seizure threshold for control and piperine-treated mice at the indicated doses. Data points represent means ± S.E.M. from at least 4 mice (**) indicates statistically significant (p<0.01) differences.

Figure 8:
Behaviour in the Elevated Plus Maze Test for control and drug-treated mice at a concentration of 0.3 mg/kg bodyweight of the indicated piperine derivative (compare the effect of piperine- dotted bar). Bars indicate the time spent on the open arms (OA) in % of the total time. Bars represent means ± S.E.M. from at least 8 different mice (*) indicates statistically significant (p<0.05) differences.

Figure 9:
Behaviour in the Elevated Plus Maze Test for control and SCT-64-treated mice at the indicated doses. Bars indicate **(A)** the time spent on the open arms (OA) in % of the total time and in **(B)** the total distance travelled, respectively. Bars represent means ± S.E.M. from at least 8 different mice (*) indicates statistically significant (p<0.05) differences.

Figure 10:
Behaviour in the Elevated Plus Maze Test for control and SCT-66-treated mice at the indicated doses. Bars indicate **(A)** the time spent on the open arms (OA) in % of the total time and in **(B)** the total distance travelled, respectively. Bars represent means ± S.E.M. from at least 8 different mice (*) indicates statistically significant (p<0.05) differences.

Figure 11:
Behaviour in the Open Field Test 30 min after i.p. injection of the indicated compound compared to control mice. Bars indicate the total ambulation upon **(A)** 10 mg/kg bodyweight and **(B)** 30 mg/kg bodyweight, respectively. Bars represent means ± S.E.M. from at least 8 mice (*) indicates statistically significant (p<0.05) differences.

Figure 12:

Effects on seizure threshold in mice upon application of PTZ 30 min after i.p injection of piperine and the indicated derivatives indicated in the figure. Data points represent mean ± SEM.

Effects on seizure threshold in mice upon application of PTZ 30 min after i.p injection of piperine and the indicated derivatives indicated in the figure. SCT-64, SCT-54 and SCT-29 were particularly active at low concentrations. Data points represent mean ± SEM.

**General synthetic procedures**

**Formation of piperic acid**

Piperine was dissolved in 2 N ethanolic KOH and refluxed for 3 days. Afterwards the reaction mixture was evaporated. The residue was dissolved in ice-water and acidulated with 2 N HCl. The precipitate was filtered, excessively washed with water and dried. The resulting crude product was crystallized from ethanol to give piperic acid as yellow crystals (melting point (mp.) 219-220°C).

**Formation of acid chloride**

A solution of piperic acid in dry tetrahydrofuran (THF) and 2.5 equivalent (eq.) thionyl chloride was refluxed for 3 hours. Afterwards the solution was evaporated and redissolved in dry THF for the next reaction step.

**Formation of ester derivatives**

To a solution of piperic acid chloride in dry THF, 2 eq. triethylamine and 5 eq. of the respective alcohol were added and stirred at room temperature (RT) over night. The reaction mixture was evaporated and poured in water. The resulting precipitate was filtered, washed with water and crystallized in an adequate solvent.

**Formation of amid derivatives**

To a solution of piperic acid chloride dissolved in dry THF, 3.5 eq. of the respective amine was added and stirred at RT over night. The reaction mixture was evaporated and poured in water. The resulting precipitate was filtered, washed with water and crystallized in an adequate solvent.

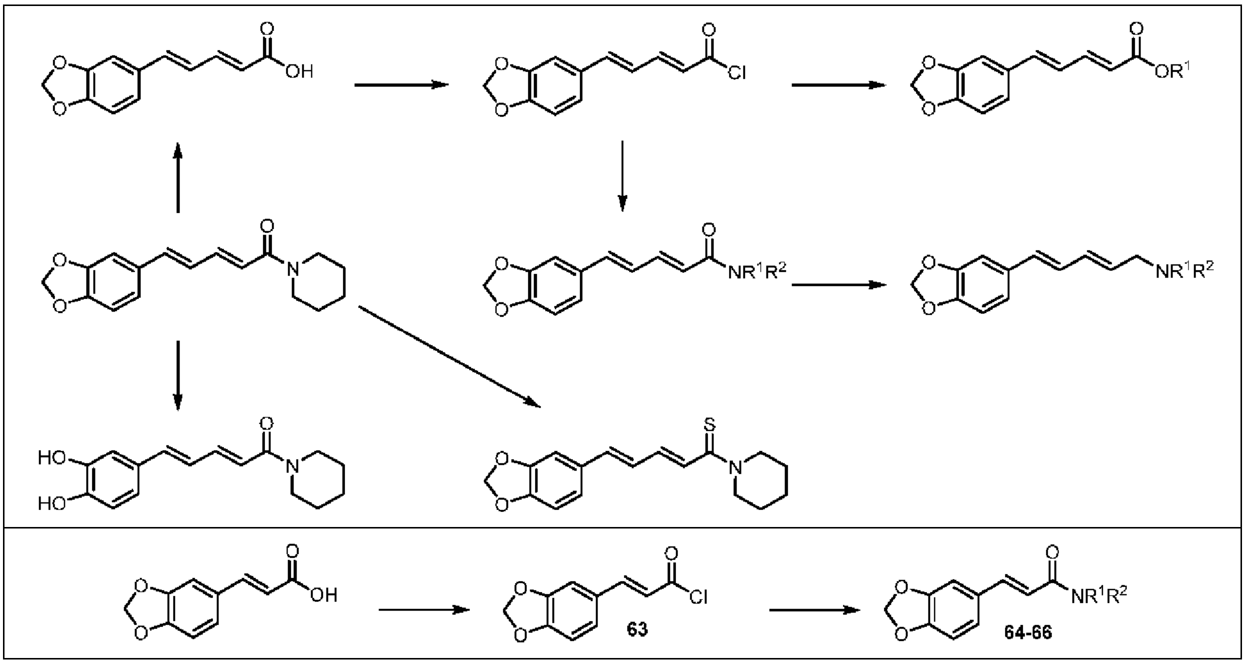

63

64-66

(continued)

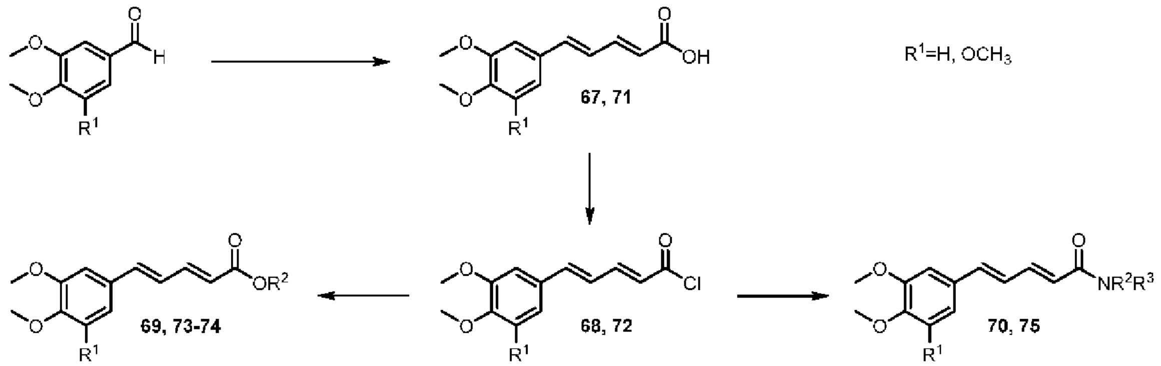


Schematic diagram of piperine syntheses

## Examples

All of the following examples 1-76 are reference examples apart from examples 34-37 and 44, which illustrate the present invention.

**Formation of (2E,4E)-5-(1,3-benzodioxol-5-yl))-*N,N*-dibutyl-2,4-pentadienamide**

To a solution of piperic acid chloride (0.710 g, 3 mmol) dibutylamine (1.357 g, 10.5 mmol) was added and stirred at RT over night. The reaction mixture was evaporated and poured in water. The resulting precipitate was filtered, washed with water and crystallized in ethanol to give slightly orange crystals (0.746 g, 75.5 %, mp. 88-90°C).

**Table 1: Examples 1 - 76**

| No | Struktur | SF | MW | mp °C | Code |
|---|---|---|---|---|---|
| 01 | 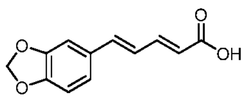 | $C_{12}H_{10}O4$ | 218.21 | 219-220 | SCT-06 |
| 02 | 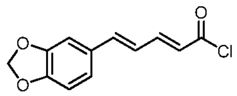 | $C_{12}H_9ClO_3$ | 236.65 | 144-148 | SCT-08 |
| 03 | 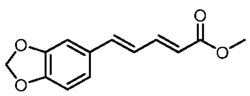 | $C_{13}H_{12}O4$ | 232.23 | 142-144 | SCT-16 |
| 04 | 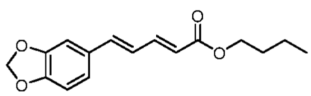 | $C_{16}H_{18}O_4$ | 274.31 | 156-160 | SCT-17 |
| 05 | 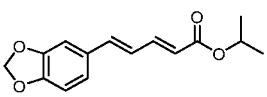 | $C_{15}H_{16}O_4$ | 260.29 | 160-163 | SCT-20 |
| 06 | 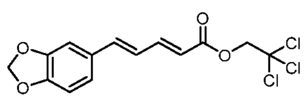 | $C_{14}H_{11}Cl_3O_4$ | 349.59 | 91-93 | SCT-27 |
| 07 | 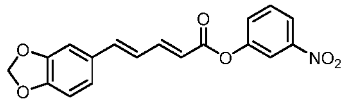 | $C_{18}H_{13}NO_6$ | 339.90 | 171-172 | SCT-35 |

(continued)

| No | Struktur | SF | MW | mp °C | Code |
|---|---|---|---|---|---|
| **08** | 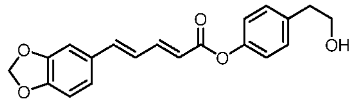 | $C_{20}H_{18}O_5$ | 338.35 | 119-121 | SCT-36 |
| **09** | 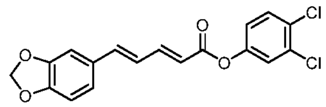 | $C_{18}H_{12}Cl_2O_4$ | 363.19 | 145-146 | SCT-37 |
| **11** | 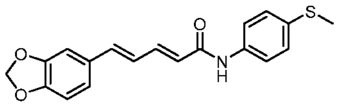 | $C_{19}H_{17}NO_3S$ | 339.41 | 200-201 | SCT-11 |
| **12** | 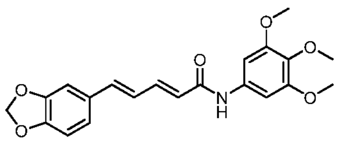 | $C_{21}H_{21}NO_6$ | 383.39 | 153-156 | SCT-13 |
| **13** | 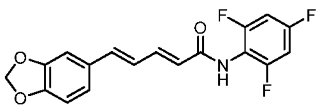 | $C_{18}H_{12}F_3NO_3$ | 347.29 | 234-235 | SCT-14 |
| **14** | 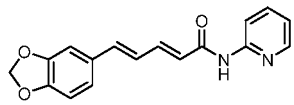 | $C_{17}H_{14}N_2O_3$ | 294.30 | 208-209 | SCT-30 |
| **15** | 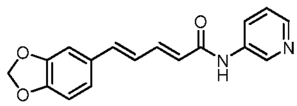 | $C_{17}H_{14}N_2O_3$ | 294.30 | 209-210 | SCT-31 |
| **16** | 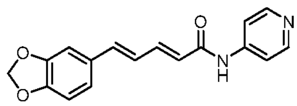 | $C_{17}H_{14}N_2O_3$ | 294.30 | 208-209 | SCT-32 |
| **17** | 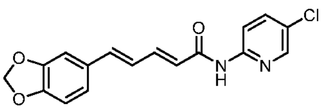 | $C_{17}H_{13}ClN_2O_3$ | 328.75 | 216-218 | SCT-33 |
| **18** | 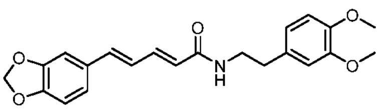 | $C_{22}H_{23}NO_5$ | 381.42 | 185-188 | SCT-28 |
| **19** | 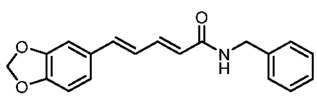 | $C_{19}H_{17}NO_3$ | 307.34 | 180-182 | SCT-47 |
| **20** | 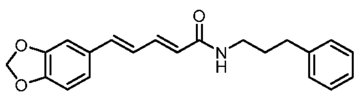 | $C_{21}H_{21}NO_3$ | 335.40 | 161-162 | SCT-34 |
| **21** | 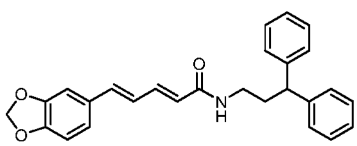 | $C_{27}H_{25}NO_3$ | 411.49 | 125-127 | SCT-43 |

(continued)

| No | Struktur | SF | MW | mp °C | Code |
|---|---|---|---|---|---|
| 22 | 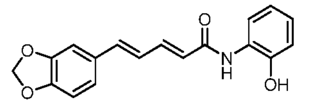 | $C_{18}H_{15}NO_4$ | 309.32 | 192-193 | SCT-39 |
| 23 | 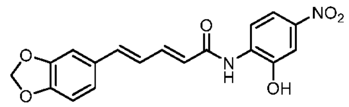 | $C_{18}H_{14}N_2O_6$ | 354.31 | 284-286 | SCT-40 |
| 24 | 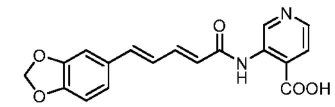 | $C_{18}H_{14}N_2O_5$ | 338.31 | 297-299 | SCT-41 |
| 25 | 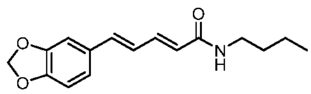 | $C_{16}H_{19}NO_3$ | 273.33 | 141-143 | SCT-42 |
| 26 | 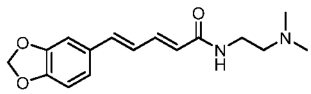 | $C_{16}H_{20}N_2O_3$ | 288.34 | 131 | SCT-21 |
| 27 | 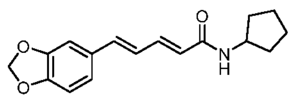 | $C_{17}H_{19}NO_3$ | 285.34 | 189-190 | SCT-44 |
| 28 | 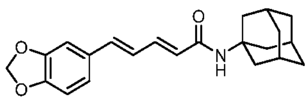 | $C_{22}H_{25}NO_3$ | 351.44 | 156-158 | SCT-48 |
| 29 | 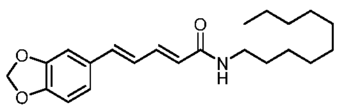 | $C_{22}H_{31}NO_3$ | 357.49 | 102-105 | SCT-51 |
| 30 | 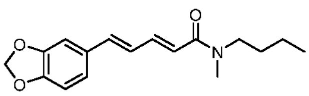 | $C_{17}H_{21}NO_3$ | 287.35 | n.t. | SCT-57 |
| 31 | 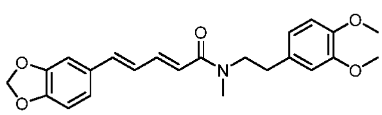 | $C_{23}H_{25}NO_5$ | 395.45 | n.t. | SCT-26 |
| 32 | 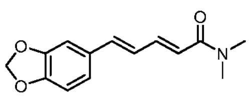 | $C_{15}H_{15}NO_3$ | 245.27 | 148-150 | SCT-53 |
| 33 | 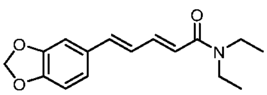 | $C_{16}H_{19}NO_3$ | 273.33 | 87-88 | SCT-54 |
| 34 | 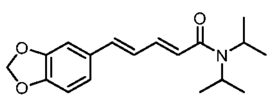 | $C_{18}H_{23}NO_3$ | 301.38 | 80-82 | SCT-46 |
| 35 | 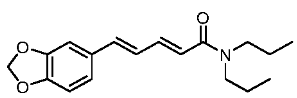 | $C_{18}H_{23}NO_3$ | 301.38 | 59-62 | SCT-64 |

(continued)

| No | Struktur | SF | MW | mp °C | Code |
|---|---|---|---|---|---|
| **36** | 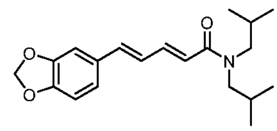 | $C_{20}H_{27}NO_3$ | 329.43 | oil | SCT-66 |
| **37** | 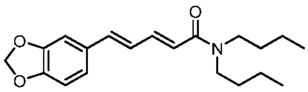 | $C_{20}H_{27}NO_3$ | 329.43 | 88-90 | SCT-29 |
| **38** | 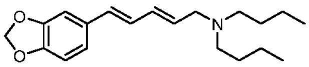 | $C_{20}H_{29}NO_2$ | 315.45 | n.t. | SCT-62 |
| **39** | 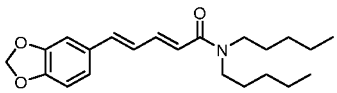 | $C_{22}H_{31}NO_3$ | 357.49 | oil | SCT-65 |
| **40** | 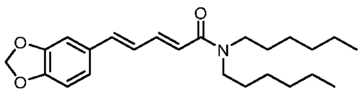 | $C_{24}H_{35}NO_3$ | 385.54 | 49-50 | SCT-52 |
| **41** | 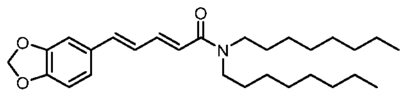 | $C_{28}H_{43}NO_3$ | 441.65 | n.t. | SCT-69 |
| **42** | 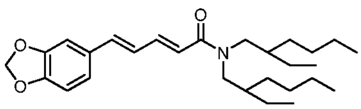 | $C_{28}H_{43}NO_3$ | 441.65 | n.t. | SCT-67 |
| **43** | 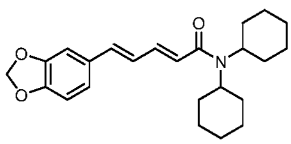 | $C_{24}H_{31}NO_3$ | 381.51 | 133-134 | SCT-50 |
| **44** | 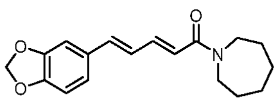 | $C_{18}H_{21}NO_3$ | 299.36 | 120-121 | SCT-18 |
| **45** | 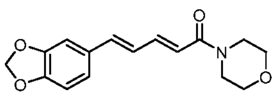 | $C_{16}H_{17}NO_4$ | 287.31 | 176-179 | SCT-09 |
| **46** | 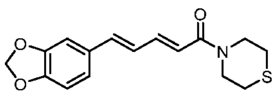 | $C_{16}H_{17}NO_3S$ | 303.38 | 125-127 | SCT-12 |
| **47** | 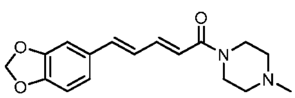 | $C_{17}H_{20}N_2O_3$ | 300.35 | 140-141 | SCT-81 |
| **48** | 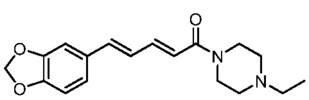 | $C_{18}H_{22}N_2O_3$ | 314.38 | 145-146 | SCT-10 |
| **49** | 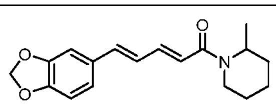 | $C_{18}H_{21}NO_3$ | 299.36 | 96-97 | SCT-73 |

(continued)

| No | Struktur | SF | MW | mp °C | Code |
|---|---|---|---|---|---|
| **50** | 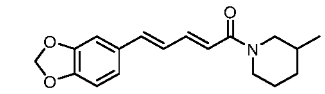 | $C_{18}H_{21}NO_3$ | 299.36 | 104-106 | SCT-74 |
| **51** | 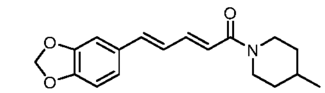 | $C_{18}H_{21}NO_3$ | 299.36 | 80-83 | SCT-75 |
| **52** | 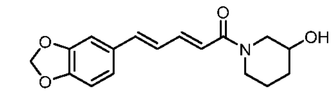 | $C_{17}H_{19}NO_4$ | 301.34 | 146-147 | SCT-76 |
| **53** | 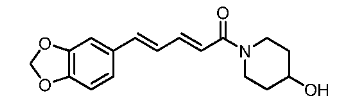 | $C_{17}H_{19}NO_4$ | 301.34 | 170 | SCT-77 |
| **54** | 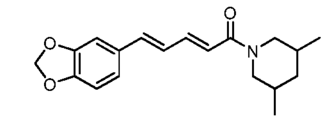 | $C_{19}H_{23}NO_3$ | 313.39 | 104-106 | SCT-78 |
| **55** | 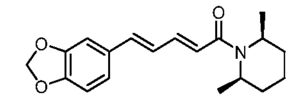 | $C_{19}H_{23}NO_3$ | 313.39 | 90-93 | SCT-79 |
| **56** | 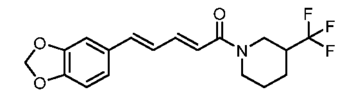 | $C_{18}H_{18}F_3NO_3$ | 353.34 | 140 | SCT-80 |
| **57** | 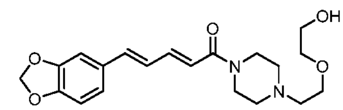 | $C_{20}H_{26}N_2O_5$ | 374.43 | 75-78 | SCT-24 |
| **58** | 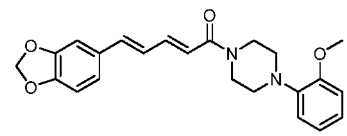 | $C_{23}H_{24}N_2O_4$ | 392.45 | 199-202 | SCT-25 |
| **59** | 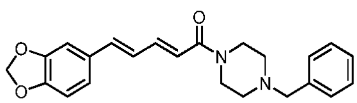 | $C_{23}H_{24}N_2O_3$ | 376.45 | 81-84 | SCT-38 |
| **60** | 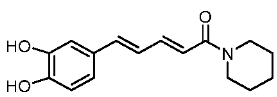 | $C_{16}H_{19}NO_3$ | 273.33 | n.t. | SCT-45 |
| **61** | 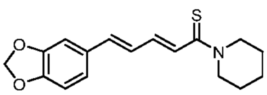 | $C_{17}H_{19}NO_2S$ | 301.40 | 177-178 | SCT-15 |
| **62** | 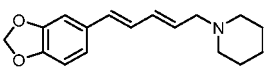 | $C_{17}H_{21}NO_2$ | 271.35 | 124-128 | SCT-63 |
| **67** | 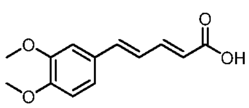 | $C_{13}H_{14}O_4$ | 234.25 | 205-207 | SCT-01 |

(continued)

| No | Struktur | SF | MW | mp °C | Code |
|---|---|---|---|---|---|
| **68** | 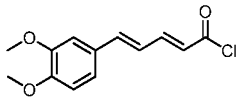 | $C_{13}H_{13}ClO_3$ | 252.69 | n.t. | SCT-04 |
| **69** | 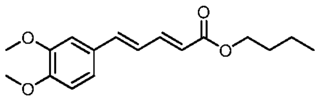 | $C_{17}H_{22}O_4$ | 290.35 | n.t. | SCT-23 |
| **70** | 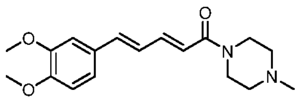 | $C_{18}H_{24}N_2O_3$ | 316.39 | n.t. | SCT-05 |
| **71** | 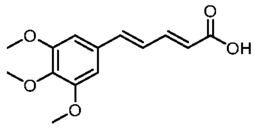 | $C_{14}H_{16}O_5$ | 264.27 | 160-162 | SCT-03 |
| **72** | 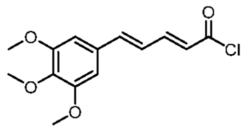 | $C_{14}H_{15}ClO_4$ | 282.72 | n.t. | SCT-56 |
| **73** | 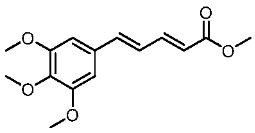 | $C_{15}H_{18}O_5$ | 278.30 | 109-111 | SCT-07 |
| **74** | 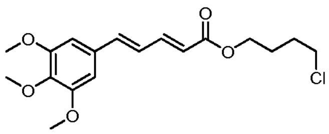 | $C_{18}H_{23}ClO_5$ | 354.83 | 64-65 | SCT-55 |
| **75** | 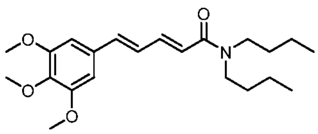 | $C_{22}H_{33}NO_4$ | 375.50 | oil | SCT-68 |
| **76** | 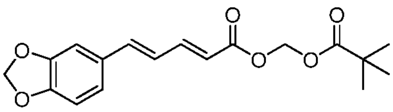 | $C_{18}H_{20}O_6$ | 332.35 | | |

## MATERIALS AND METHODS

### Piperine

Piperine was isolated from Piper nigrum as described by Zaugg et al. (2010) and obtained from SigmaAldrich (Vienna, Austria), respectively. Piperine derivatives were synthesized as described above. Stock solutions (1mg/10 µl) were prepared in DMSO (Dimethyl Sulfoxide, Sigma, Austria).Working concentrations were adjusted by dilution with 0.9% sodiumchloride. pH was adjusted to 7.2-7.4 with NaOH. For better solubility 3% of Tween80 was added to all solutions. All solutions were freshly prepared every day prior to experiments. Care was taken for equal amounts of DMSO in the actein and control solutions.

## Animals

Male mice (C57Bl/6N) were obtained from Charles River Laboratories (Germany). For breeding and maintenance mice were group housed with free access to food and water. Temperature was fixed to 23±1 °C and 60% humidity with a 12 h light-dark cycle (lights on 0700-1900 hours). Male mice at 3-6 months age were tested in all experiments. Every effort was taken to minimize the number of animals used (Khom et al., 2010).

## Behavioural Experiments

## Open Field test

Ambulation was tested over 10 min in a 50x50 cm flexfield box equipped with infrared rearing detection. Illumination was set to 150 lux. Animals were video monitored and their explorative behavior was analyzed using the Actimot 2 equipment and software (TSE-systems, Bad Homburg, Germany). Arenas were subdivided into border (up to 8 cm from wall), center (20x20 cm, i.e. 16% of total area), and intermediate area according to the recommendations of EMPRESS (European Mouse Phenotyping Resource of Standardised Screens; http://empress.har.mrc.ac.uk).

## Elevated Plus Maze test (EPM)

The animals's behavior was tested over 5 min on an elevated plus maze 1 m above ground consisting of two closed and two open arms, each 50x5 cm in size (Khom et al., 2010). The test instrument was built from gray PVC; the height of closed arm walls was 20 cm. Illumination was set to 180 lux. Animals were placed in the center, facing an open arm. Analysis of open and closed arm entries and time on open arm was automatically done with Video-Mot 2 equipment and software (Khom et al., 2010).

## *Seizure threshold*

Seizure threshold was determined by PTZ tail-vein infusion on freely moving animals at a rate of 100 μl/min (100 μg/ml PTZ in saline, pH 7.4). Infusion was stopped when animals displayed generalized clonic seizures. Animals were immediately killed by cervical displacement after the first generalized seizure. The seizure threshold dose was calculated from the infused volume in relation to body weight (Loacker et al. 2007). Piperine and piperine derivatives were applied at doses from 0.1 to 30 mg/kg 30 min before PTZ infusion. Control animals were pretreated with 10 % DMSO in saline containing 3 % Tween 80 (i. e. the solvent for all piperine doses).

## Characterisation of the modulation of $GABA_A$ receptors by piperine and its derivatives

The following paragraphs describe the preparation of oocytes expressing the specific $GABA_A$ receptor subtype composed of $\alpha_1$, $\beta_2$ and $\gamma_2$ subunits and the subsequent analysis of the concentration-response curves of the stimulation of chloride currents through $GABA_A$ receptors ($I_{GABA}$).

Preparation of stage V-VI oocytes from *Xenopus laevis,* synthesis of capped off run-off poly($A^+$) cRNA transcripts from linearized cDNA templates (pCMV vector) was performed as described (Khom et al. 2006). Briefly, female *Xenopus laevis* (NASCO, USA) were anaesthetised by exposing them for 15 minutes to a 0.2 % solution of MS-222 (methane sulfonate salt of 3-aminobenzoic acid ethyl ester; Sandoz) before surgically removing parts of the ovaries. Follicle membranes from isolated oocytes were enzymatically digested with 2 mg/ml collagenase (Type 1A, Sigma). One day after isolation, the oocytes were injected with about 10-50 μl of DEPC- treated water (diethyl pyrocarbonate, Sigma, Germany) containing the different cRNAs (encoding for $\alpha_1$, $\beta_2$ and $\gamma_2$ subunits) at a concentration of approximately 300 - 3000 pg/μl/subunit. The amount of cRNA was determined by means of a NanoDrop ND-1000 (Kiskerbiotech, Steinfurt, Germany).

Oocytes were stored at 18°C in ND96 solution (Methfessel et al., 1986). Electrophysiological experiments were done using the two-microelectrode voltage-clamp method at a holding potential of -70 mV making use of a TURBO TEC 01C amplifier (npi electronic, Tamm, Germany) and an Axon Digidata 1322A interface (Molecular Devices, Sunnyvale, CA). Using pCLAMP v.9.2 data acquisition was carried out. The bath solution contained 90 mM NaCl, 1 mM KCl, 1 mM $MgCl_2 \cdot 6H_2O$, 1 mM $CaCl_2$ and 5 mM HEPES (pH 7.4). Microelectrodes were filled with 2M KCl and had resistances between 1 and 3 MΩ (Khom et al., 2007).

GABA and piperine and its derivatives, respectively, were applied by means of fast perfusion system (see Baburin et al. 2006 for details; Khom et al. 2006). Drug or control solutions were applied by means of a TECAN Miniprep 60 permitting automation of the experiments. To elicit $I_{GABA}$ the chamber was perfused with 120 μl of GABA-containing solution at volume rate between 300 and 1000 μl/s. The $I_{GABA}$ rise time ranged between 100 and 250 ms (see Khom

et al. 2006). Care was taken to account for possible slow recovery from increasing levels of desensitization in the presence of high GABA, piperine and piperin derivatives concentrations. Oocytes with maximal current amplitudes >3 $\mu$A were discarded to exclude voltage-clamp errors (Khom et al., 2006; Baburin et al., 2006; Khom et al., 2007).

Stimulation of chloride currents by modulators of the $GABA_A$ receptor was measured at a GABA concentration eliciting between 5 and 10 % of the maximal current amplitude ($EC_{5-10}$). The $EC_{5-10}$ was determined at the beginning of each experiment.

Enhancement of the chloride current was defined as $(I_{(GABA+Comp)}/I_{GABA}) - 1$, where $I_{(GABA+Comp)}$ is the current response in the presence of a given compound (piperine or piperin derivatives) and $I_{GABA}$ is the control GABA current. To measure the sensitivity of the $GABA_A$ receptor for a given compound, it was applied for an equilibration period of 1 minute before applying GABA ($EC_{5-10}$). Concentration-response curves were generated and the data were fitted by non-linear regression analysis using Origin software (OriginLab Corporation, USA). Data were fitted to the equation:

$$\frac{1}{1+\left(\frac{EC_{50}}{[Comp]}\right)^{n_H}},$$

where $n_H$ is the Hill coefficient. Each data point represents the mean $\pm$ S.E. from at least 4 oocytes and $\geq$ 2 oocyte batches. Statistical significance was calculated using paired Student *t*-test with a confidence interval of $p < 0.05$ (Khom et al., 2007).

**Modulation of $I_{GABA}$ by piperine and its derivatives**

The modulation of $I_{GABA}$ by piperine and piperine derivatives was investigated in a system as outlined above, i.e. on recombinant $GABA_A$ receptors expressed in *Xenopus laevis* oocytes on $GABA_A$ channels composed of $\alpha_1\beta_2\gamma_2$ subunits. Piperine and piperine derivatives were diluted in DMSO; however, the maximum DMSO concentration in the bath (0.3 %) did not affect $I_{GABA}$ (Khom et al., 2006; Khom et al., 2007).

As shown in Fig. 2 A-C, piperine and the derivatives SCT29, SCT64 and SCT66 induced a positive allosteric modulatory effect by enhancing $I_{GABA}$. Each data point in Fig. 2 A-C represents means $\pm$ S.E.M. from at least 2-7 oocytes from $\geq$ 2 batches.

**Characterisation of the modulation of the human vaniloid receptor (TRPV1) by piperine and its derivatives**

cRNA encoding for the human TRPV1 receptor (hTRPV1) was injected into Xenopus oocytes as previously described (Hu et al. 2004). As shown in Figure 4 piperine (300 $\mu$M) but none of the derivatives SCT29, SCT64 or SCT66 induced cation currents through TRPV1 receptors when applied at a concentration of 300 $\mu$M.

***In vivo* effects of Piperine and derivatives**

**Sedative and anxiolytic effects of piperine**

The effect of piperine on anxiety-related behavior was tested 30 min after i. p. injection in the Elevated Plus Maze test (EPM). As illustrated in Figure 5A, control mice (treated with saline) spent 30.3 $\pm$ 3.4, n=21 in the open arms of the EPM. At a dose of 0.1 mg/kg bodyweight no significant effect was observed. At higher doses (i.e. 0.3 and 1 mg/kg bodyweight) mice spent significantly ($p<0.05$) more time in the open arms compared to control mice (0.3 mg/kg bodyweight: 42.7 $\pm$ 4.4, n=21 and 1 mg/kg bodyweight: 45.6 $\pm$ 6.6, n=16). However, a trend towards a slightly reduced distance was also observed (see Figure 5B). Thus, higher doses were not tested in the EPM.

The EPM test is a standard in drug discovery processes to detect anxiolytic properties as it is based on the innate fear of mice to spend time on open, elevated spaces (e.g. the open arms), thus they tend to avoid these areas. In the EPM test, the time spent in the open arms is consequently a reliable measure of anxiety-reducing effects of novel compounds. The data clearly show, that piperine - as shown in Fig.5A - reveals strong anxiolytic effects.

***Sedation***

In the EPM test, at doses $\geq$1 mg/kg bodyweight of piperine, mice covered a slightly reduced distance compared to control littermates indicating sedative effects of piperine. Thus, the effects of higher doses of piperine in the Open Field Test (OF) were studied. As illustrated in Fig.6, a traveled distance of approx. 40 m for control mice (i.e. treated

with vehicle; 37.4 ± 1.6, n=10) was measured. i.p. injection of piperine resulted in a dose-dependent reduction of the traveled distance. At a dose of 30 mg/kg bodyweight, ambulation was reduced more than 50% compared to control littermates (control: 37.4 ± 1.6, n=10 vs. 30 mg/kg bodyweight 16.5 ±3.8 m; n=10; p<0.05).

In the OF test both drug-induced effects on anxiety-related behavior and drug-induced sedation can be analyzed. It combines the aversive stimulus of an open space and therefore an appropriate-sized area to detect also differences in ambulation. Commonly, a reduction of ambulation is associated with sedation, in contrast to an increased ambulation meaning hyperactivity.

Mice treated with piperine displayed at all doses studied - see Fig.6- less ambulation compared to control animals indicating that piperine at higher dose (i.e. ≥ 3 mg/kg bodyweight; p<0.05) induces strong sedative effects.

***Anticonvulsant (Antiepileptic) effects* - *Analysis of the seizure threshold***

The seizure threshold as assessed by pentylentetrazole (PTZ) tail vein infusions was significantly increased 30 min after i-p. injection of 3 or 10 mg piperine/kg (46.2 ± 5.44; n=4 and 48.7 ± 2.13; n=4 for 3 and 10 mg/kg respectively vs. 39.4 ± 2.83; n=7 for DMSO/saline controls). A dose of 30 mg/kg resulted in a significant drop in seizure threshold (30.3 ± 3.43; n= 4; Fig.3). Doses below 3 mg/kg did not affect seizure threshold. At the infusion rate of 100 µl/min generalized seizures are induced in less than 2 min after infusion of PTZ was started.

The effects of piperine on the PTZ induced seizure threshold revealed a biphasic dose response curve. At doses of 1 mg/kg bodyweight increased thresholds were observed, which suddenly dropped to decreased thresholds at a dose of 30 mg/kg. This suggest activation of at least one other receptor type than the $GABA_A$ receptor e.g. TRPV1 channels.

***In vivo* effects of Piperine derivatives**

After the identification of piperine as novel anxiolytic, anticonvulsant and sedative GABAA receptor ligand, first *in vivo* effects of the most promising derivatives (SCT-29 (Ex 37), SCT-64 (Ex 35) and SCT-66 (Ex 36)) were studied in the EPM.

As illustrated in Fig.8, i.p. injection of compounds SCT-64 and SCT-66 at a dose of 0.3 mg/kg bodyweight resulted in a significantly increased time spent on the open arms of the EPM compared to control littermates (Saline 31.5 ± 2.6; n= 13 vs. SCT-64 41.6 ± 3.2; n=13 vs. SCT-66 40.8 ± 3.2; n=14; p<0.05), while a trend towards more time spent in the open arms of the EPM was observed for SCT-29, that was, however, not statistically significant at the tested doses. The effects of SCT-64 and SCT-66 were, thus, studied in more detail.

**Anxiolytic effects of SCT-64**

As illustrated in Fig. 9A, mice treated with doses ≥ 0.3 mg/kg bodyweight SCT-64 spent significantly more time in the open arms of the EPM compared to control mice (Control: 35.3 ± 4.2; n= 12 vs. 0.3 mg/kg bodyweight SCT-64: 44.2 ± 3.3; n=18; p<0.05). This effect was dose-dependent and reached a maximum at a dose of 10 mg/kg bodyweight (59.6 ± 4.9; n= 14). As illustrated in Fig. 9B, SCT-64 also showed an on locomotor activity reducing slightly the total ambulation at doses ≥1 mg/kg bodyweight.

**Anxiolytic effects of SCT-66**

Similar effects were observed for SCT-66 in the EPM test. As with SCT-64, mice treated with doses ≥ 0.3 mg/kg bodyweight SCT-66 spent significantly more time in the open arms of the EPM compared to control mice (Control: 26.8 ± 4.2; n= 14 vs. 0.3 mg/kg bodyweight SCT-66: 38.1 ± 5.1; n=21; p<0.05). This effect was dose-dependent and reached a maximum at a dose of 3 mg/kg bodyweight (46.5 ± 6.6; n= 14; see Fig. 10A).

However, in contrast to SCT-64 no significant effect on ambulation induced by SCT-66 was observed in the EPM test (see Fig. 10B).

**Sedation**

A more detailed study on the effect of piperine derivatives SCT-29, SCT-54 (Ex 33), SCT-64 and SCT-66 was performed in the OF test.

As shown in Fig. 11A, there was not any significant effect on the total ambulation after injection of any of the piperine derivatives observed, while a reduction of the total distance in the OF - as mentioned above- after administration of piperine was obvious. However, at higher doses (see Fig. 11 B) 30 mg/kg bodyweight SCT-64 and SCT-66 reduced the total distance in the OF test, while still no effect on ambulation was observed for compounds SCT-29 and SCT-54

indicating that these two compounds lack sedative effects.

These data suggest that derivatisation of piperine results in both stronger anxiolytic and reduced sedative effects.

### Anticonvulsant (Antiepileptic) effects

As illustrated in Fig. 12 some piperine derivatives (for better comparison the effect of piperine is illustrated as black line) exhibited strong antiepileptic effects on PTZ-induced seizures and increased significantly seizure threshold. However, we observed remarkable differences between the derivatives: while for piperine derivative SCT-66 first significant effects on seizure threshold were observed at doses ≥10 mg/kg bodyweight, the other compounds exhibited their strongest effects on seizure threshold at doses between 0.1 and 3 mg/kg bodyweight.

### Concluding remarks

In the *in vivo* studies, for the first time evidence for the anxiolytic and sedative properties of piperine is provided. Moreover, SCT-64 and SCT-66 were identified as novel anxiolytic compounds with significantly reduced sedative properties. Interestingly, in this study we have also identified the first piperine derivatives (particularly SCT-64, and SCT-29, and at higher concentrations also SCT-66) as novel antiepileptic compounds lacking sedative effects. Sedation represents a major problem in the conventional pharmacotherapy of epilepsy, thus the search for compounds lacking sedative effects is a challenging goal in drug discovery.

The data on selected piperine derivatives suggest a potential of these compounds as novel antiepileptics and anxiolytics.

### References:

Johnston, G. A. R.; Hanrahan, J. R.; Chebib, M.; Duke, R. K.; Mewett, K. N. Adv. Pharmacol. 2006, 54, 286-316.
Tsang, S. Y.; Xue, H. Curr. Pharm. Design 2004, 10, 1035-1044.
Pedersen, M. E.; Metzler, B.; Stafford, G. I.; van Staden, J.; Jaeger, A. K.; Rasmussen, H. B. Molecules 2009, 14, 3833-3843.
Pei, Y. Q. Epilepsia 1983, 24, 177-182.
Szallasi, A. Trends in Pharmacological Sciences 2005, 26, 437-439.
Sunila, E.; Kuttan, G. J. Ethnopharmacol 2004, 90, 339-346.
Whiting, P. J., Curr. Opin. Pharmacol. 2006, 6, 24-29.
Kaplan, E. M.; DuPont, R. L. Curr. Med. Res. Opin. 2005, 21, 941-950.
Rupprecht, R.; Eser, D.; Zwanzger, P.; Moeller, H.-J. World J. Biol. Psychiatry 2006, 7, 231-237.
McNamara, F. N.; Randall, A.; Gunthorpe, M. J. Brit. J. Pharmacol. 2005, 144, 781 - 790.
Anand, P.; Kunnumakkara, A. B.; Newman, R. A.; Aggarwal, B. B. Mol. Pharm. 2007, 4, 807-818.
Baburin, I.; Beyl, S.; Hering, S. Pflug. Arch. Eur. J. Phy. 2006, 453, 117-123.
Khom, S.; Baburin, I.; Timin, E. N.; Hohaus, A.; Sieghart, W.; Hering, S. Mol. Pharmacol. 2006, 69, 640-649.
Khom S., Baburin I., Timin E., Hohaus A., Trauner G., Kopp B., Hering S. Neuropharmacology. 2007, 53(1):178-87.
Hu HZ, Gu Q, Wang C, Colton CK, Tang J, Kinoshita-Kawada M, Lee LY, Wood JD, Zhu MX. J Biol Chem. 2004 Aug 20;279(34):35741-8.
Zaugg J., Baburin I., Strommer B., Kim H.-J-, Hering S. Hamburger M. J Nat Prod. 2010,73(2):185-91
Khom S, Strommer B, Ramharter J, Schwarz T, Schwarzer C, Erker T, Ecker GF, Mulzer J, Hering S. Br J Pharmacol. 2010, Sep;161(1):65-78
Loacker S, Sayyah M, Wittmann W, Herzog H, Schwarzer C. Brain. 2007. Apr;130(Pt 4):1017-28. Epub 2007 Mar 8

## Claims

**1.** A compound selected from the group consisting of

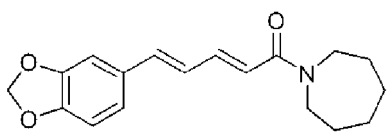,
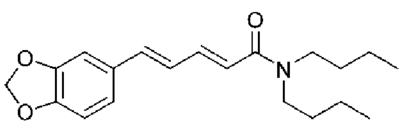,

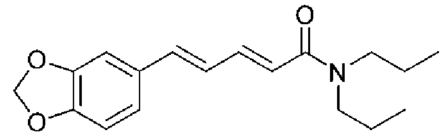 and 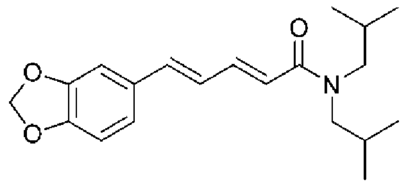

for use as a medicament.

2. A compound selected from the group consisting of

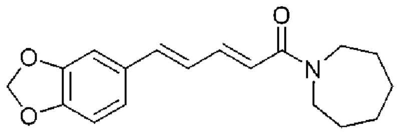, 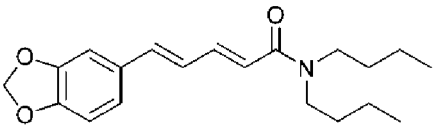,

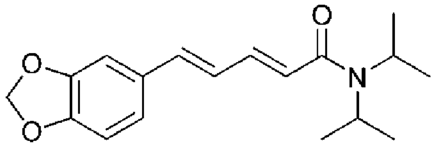, 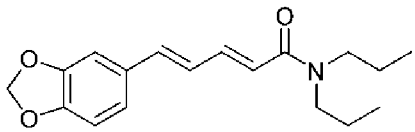

and

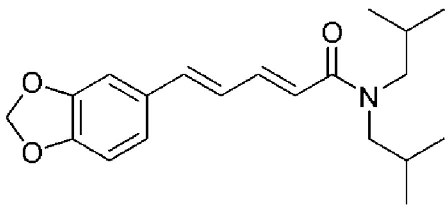

for use in modulating a pharmacological response from the $GABA_A$ receptor.

3. A compound according to claim 2 for use in treating or preventing anxiety and/or insomnia, epilepsy, depression, pain, mood and panic disorders, schizophrenia and/or disorders/symptoms connected to alcohol and/or substance withdrawal/abuse.

4. A compound according to claim 2 for use in treating or preventing epilepsy or as an anticonvulsant.

5. A compound for use according to claim 4, wherein the compound is selected from the group consisting of

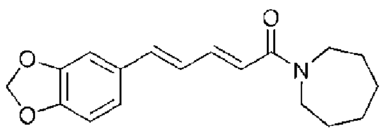, 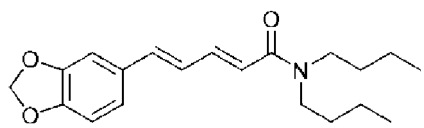,

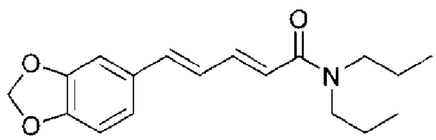 and 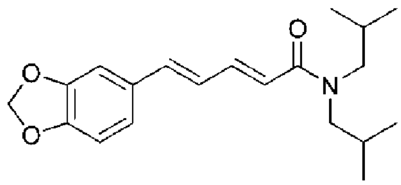.

**6.** A compound according to claim 2 for use in treating or preventing pain.

**7.** A compound for use according to claim 6, wherein the compound is selected from the group consisting of

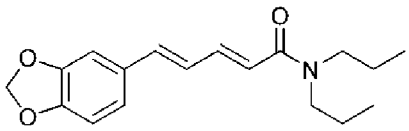 and 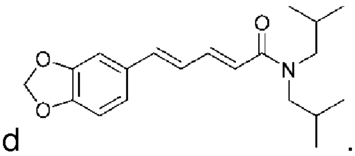.

**8.** A pharmaceutical preparation for use as a medicament, comprising one or more compounds selected from the group consisting of

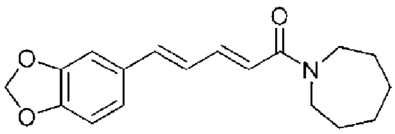, 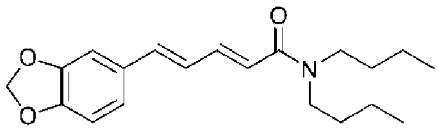,

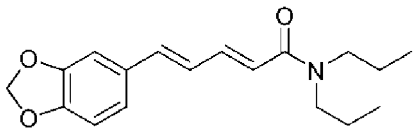 and 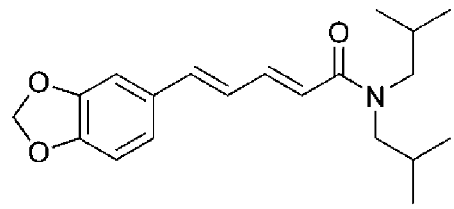

and the pharmacologically effective salts thereof, in combination with conventional excipients and/or carriers.

**9.** A pharmaceutical preparation comprising one or more compounds selected from the group consisting of

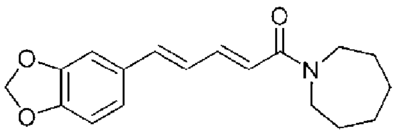, 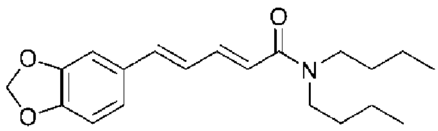,

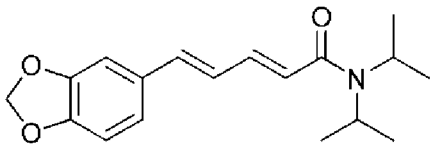, 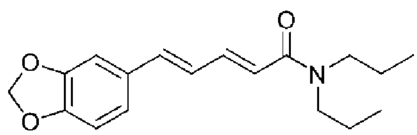

and

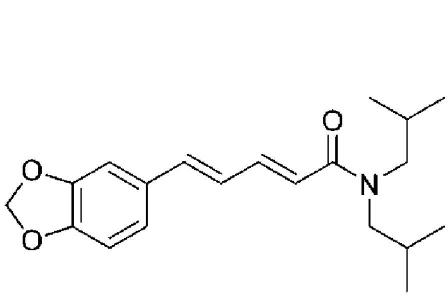

and the pharmacologically effective salts thereof, in combination with conventional excipients and/or carriers for use as an analgesic, anesthetic, sedative, hypnotic, anxiolytic, and/or antiepileptic.

**Patentansprüche**

1. Verbindung ausgewählt aus der Gruppe bestehend aus

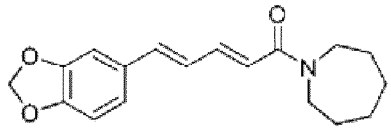, 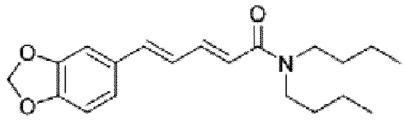,

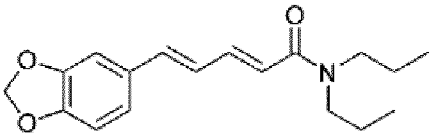 und 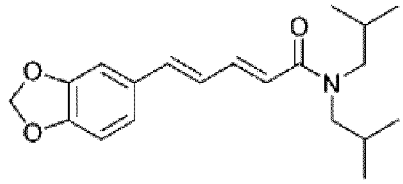

zur Verwendung als Arzneimittel.

2. Verbindung ausgewählt aus der Gruppe bestehend aus

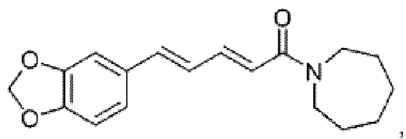, 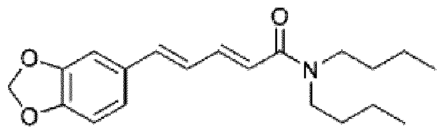,

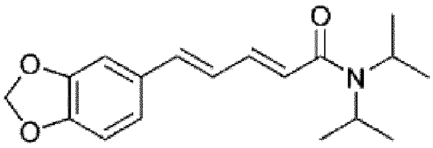, 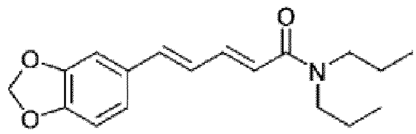

und

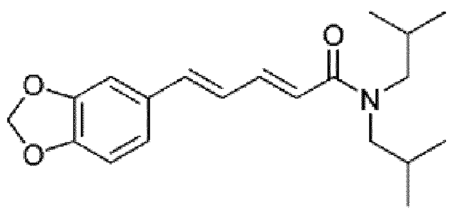

zur Verwendung zum Modulieren einer pharmakologischen Antwort des $GABA_A$ Rezeptors.

3. Verbindung nach Anspruch 2 zur Verwendung bei der Behandlung oder Vorbeugung von Angst und/oder Schlaflosigkeit, Epilepsie, Depression, Schmerz, Stimmungs- und Panikerkrankungen, Schizophrenie und/oder Erkrankungen/Symptomen im Zusammenhang mit Alkohol und/oder Drogenentwöhnung/missbrauch.

4. Verbindung nach Anspruch 2 zur Verwendung bei der Behandlung oder Vorbeugung von Epilepsie oder als krampflösendes Mittel.

5. Verbindung zur Verwendung nach Anspruch 4, wobei die Verbindung ausgewählt ist aus der Gruppe bestehend aus

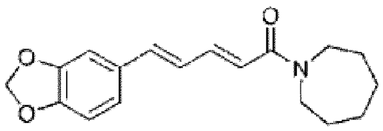, 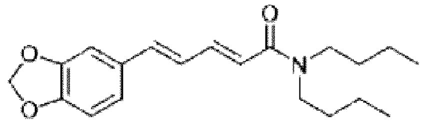,

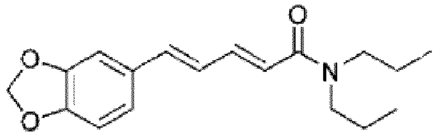 und 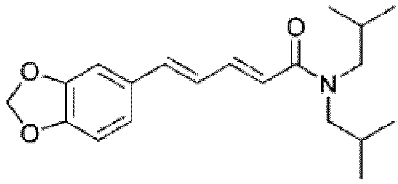.

6. Verbindung nach Anspruch 2 zur Verwendung bei der Behandlung oder Vorbeugung von Schmerz.

7. Verbindung zur Verwendung nach Anspruch 6, wobei die Verbindung ausgewählt ist aus der Gruppe bestehend aus

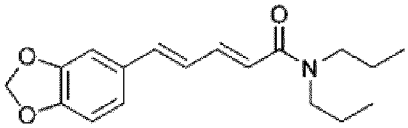 und 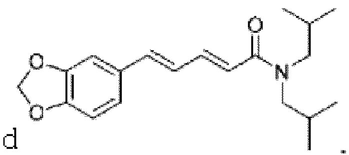.

8. Pharmazeutisches Präparat zur Verwendung als Arzneimittel, das eine oder mehrere Verbindungen umfasst, die ausgewählt sind aus der Gruppe bestehend aus

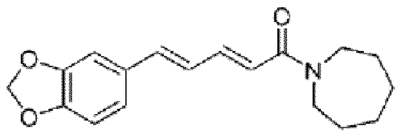, 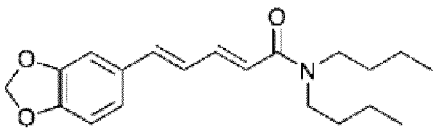,

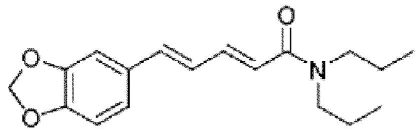 und 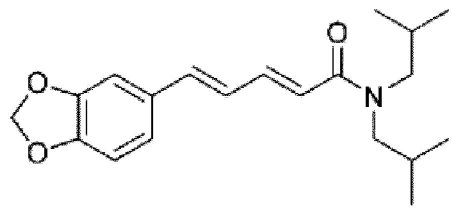

und den pharmakologisch wirksamen Salzen davon, in Kombination mit herkömmlichen Bindemitteln und/oder Trägern.

9. Pharmazeutisches Präparat, das eine oder mehrere Verbindungen umfasst, die ausgewählt sind aus der Gruppe bestehend aus

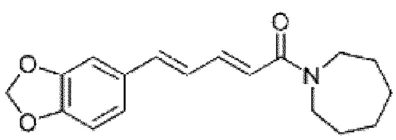, 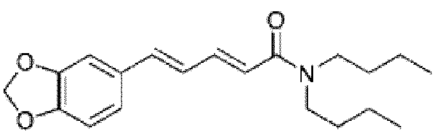,

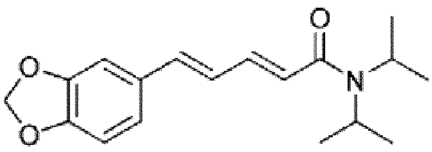
,
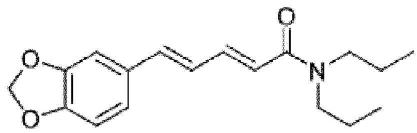

und

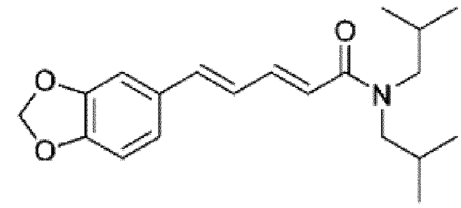

und den pharmakologisch wirksamen Salzen davon, in Kombination mit herkömmlichen Bindemitteln und/oder Trägern zur Verwendung als Analgetikum, Anästhetikum, Sedativum, Hypnotikum, Anxiolytikum und/oder Antiepileptikum.

**Revendications**

1. Composé sélectionné dans le groupe constitué de

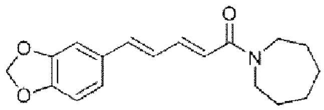
,
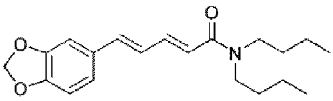
,

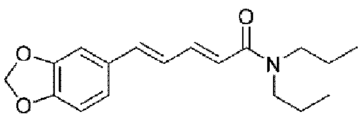
**et**
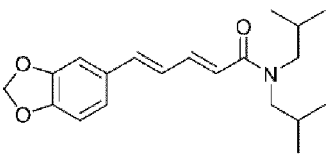

pour son utilisation en tant que médicament.

2. Composé sélectionné dans le groupe constitué de

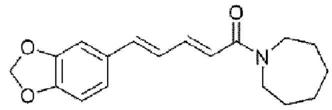
,
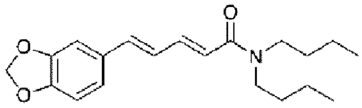
,

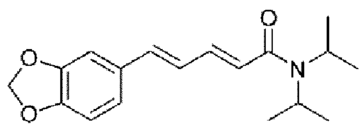
,
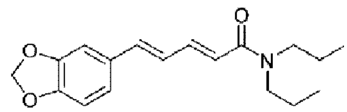

et

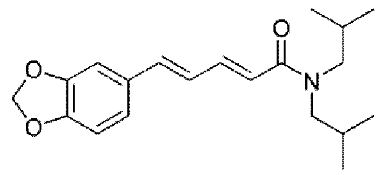

pour son utilisation pour moduler une réponse pharmacologique du récepteur $GABA_A$.

3. Composé selon la revendication 2, pour son utilisation dans le traitement ou la prévention de l'anxiété et/ou de l'insomnie, de l'épilepsie, de la dépression, de la douleur, des troubles de l'humeur et des troubles paniques, de la schizophrénie et/ou des troubles/symptômes liés au sevrage alcoolique/à l'alcoolisme et/ou au sevrage d'une substance/à la toxicomanie.

4. Composé selon la revendication 2, pour son utilisation dans le traitement ou la prévention de l'épilepsie ou en tant qu'anticonvulsivant.

5. Composé pour son utilisation selon la revendication 4, dans lequel le composé est sélectionné dans le groupe constitué de

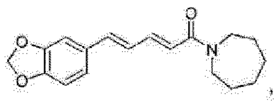,
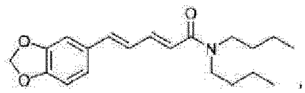,
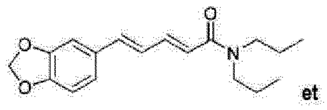
et
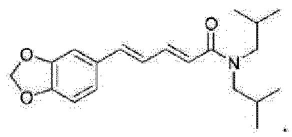.

6. Composé selon la revendication 2, pour son utilisation dans le traitement ou la prévention de la douleur.

7. Composé pour son utilisation selon la revendication 6, dans lequel le composé est sélectionné dans le groupe constitué de

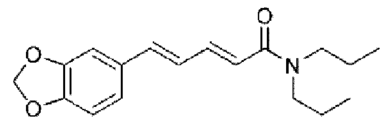
et
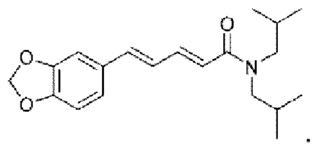.

8. Préparation pharmaceutique pour son utilisation en tant que médicament, comprenant un ou plusieurs composés sélectionnés dans le groupe constitué de

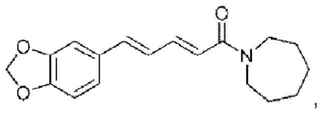,
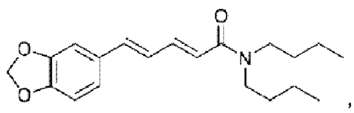,
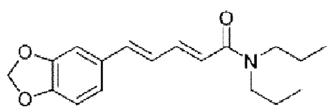
et
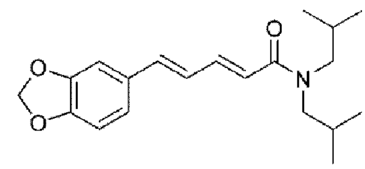

et les sels pharmacologiquement efficaces de ceux-ci, en combinaison avec des excipients et/ou des véhicules classiques.

**9.** Préparation pharmaceutique comprenant un ou plusieurs composés sélectionnés dans le groupe constitué de

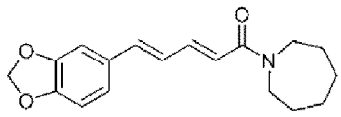

,

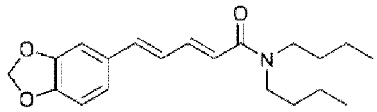

,

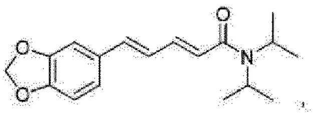

,

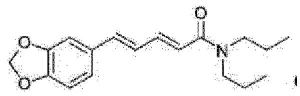

,

et

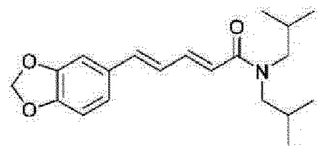

et les sels pharmacologiquement efficaces de ceux-ci, en combinaison avec des excipients et/ou des véhicules classiques pour son utilisation en tant qu'analgésique, anesthésique, sédatif, hypnotique, anxiolytique, et/ou antiépileptique.

**Figure 1:**

Modulation of $I_{GABA}$ by piperine and its derivatives

| Name | Potentiation of $I_{GABA}$ ± S.E.M. |
|---|---|
| Piperine | 226 ± 26 |
| SCT10 | 0 ± 0 |
| SCT18 | 180 ± 35 |
| SCT21 | -46 ± 2 |
| SCT24 | 0 ± 0 |
| SCT25 | 0 ± 0 |
| SCT29 | 279 ± 79 |
| SCT46 | 316 ± 42 |
| SCT47 | 0 ± 0 |
| SCT51 | 0 ± 0 |
| SCT63 | 0 ± 0 |
| SCT64 | 1174 ± 269 |
| SCT66 | 646 ± 77 |
| SCT68 | 0 ± 0 |
| SCT74 | 182 ± 52 |
| SCT80 | 55 ± 23 |
| SCT81 | -28 ± 3 |

**Figure 2**

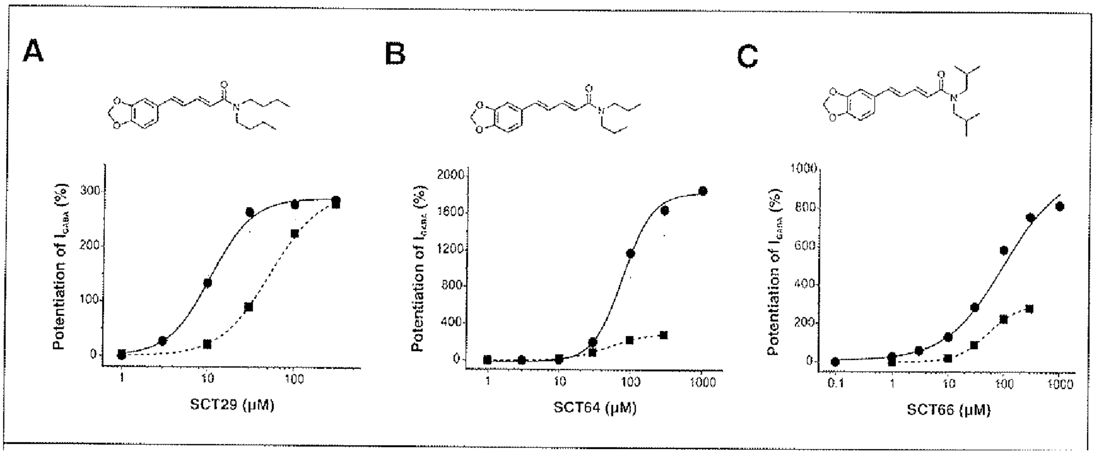


| | $EC_{50}$ [µM] | $n_H$ | max* [%] |
|---|---|---|---|
| Piperine | 52 ± 9 | 1.5 ± 0.2 | 302 ± 27 |
| SCT29 | 11 ± 2 | 1.8 ± 0.2 | 291 ± 24 |
| SCT64 | 78 ± 20 | 2.1 ± 0.6 | 1829 ±192 |
| SCT66 | 99 ± 32 | 0.8 ± 0.1 | 1003 ± 134 |

*The maximum potentiation of $I_{GABA}$ was determined through fitting the curve to the Hill equation.

**Figure 3:**

Activation of TRPV1 by piperine and its derivatives

| Name | Potentiation of TRPV1 ± S.E.M. |
|---|---|
| Piperine | 100 ± 0 |
| SCT10 | 0 ± 0 |
| SCT18 | 0 ± 0 |
| SCT21 | 0 ± 0 |
| SCT24 | 0 ± 0 |
| SCT25 | 0 ± 0 |
| SCT29 | 0 ± 0 |
| SCT46 | 0 ± 0 |
| SCT47 | 0 ± 0 |
| SCT51 | 0 ± 0 |
| SCT63 | 0 ± 0 |
| SCT64 | 0 ± 0 |
| SCT66 | 0 ± 0 |
| SCT68 | 0 ± 0 |
| SCT74 | 57 ± 18 |
| SCT80 | 0 ± 0 |
| SCT81 | 0 ± 0 |

Figure 4:

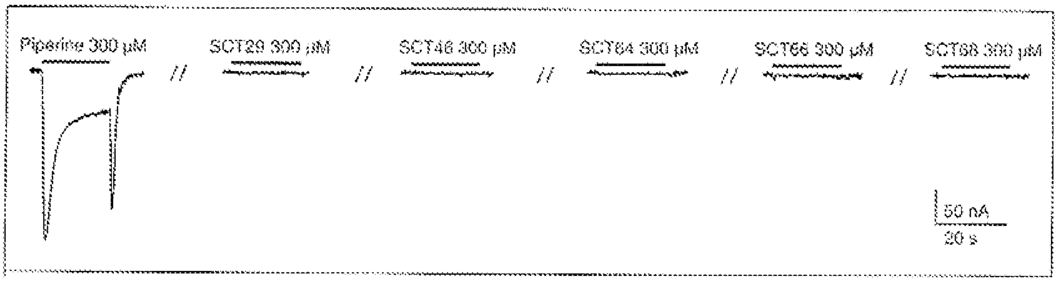


Figure 5:

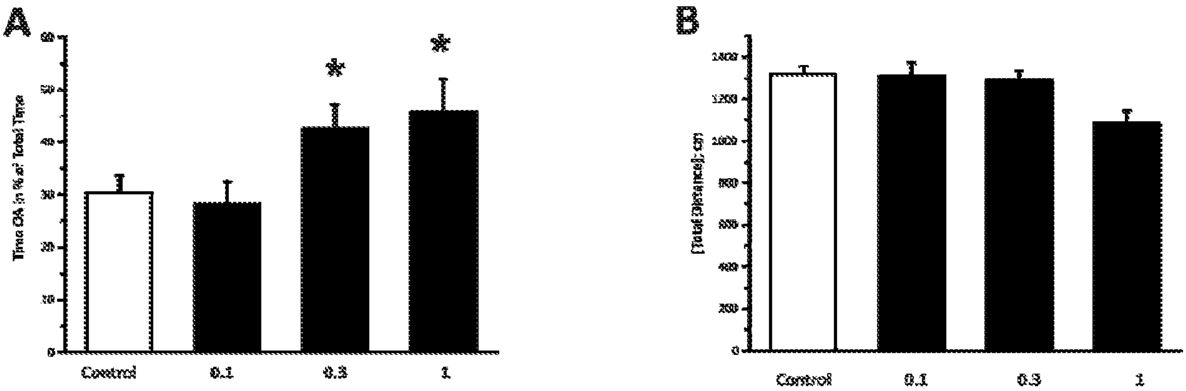


Figure 6:

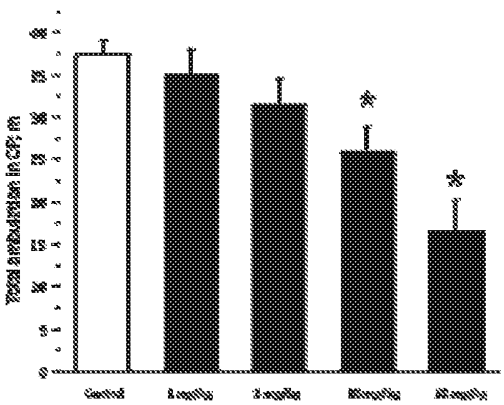

Figure 7:

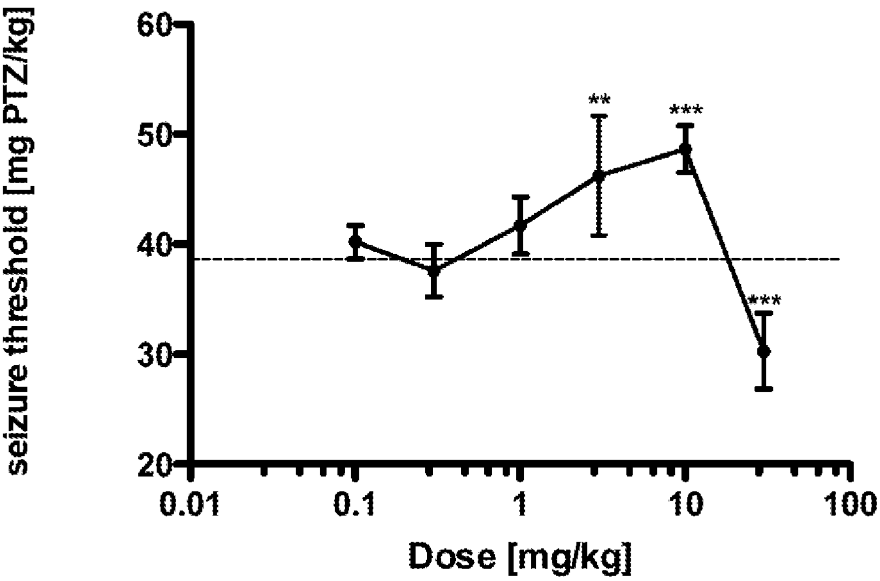


Figure 8:

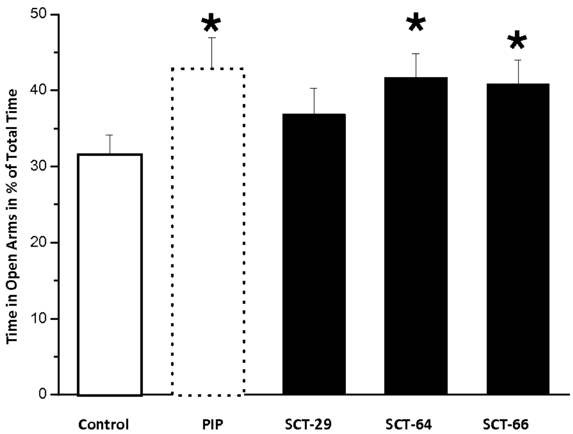

Figure 9:

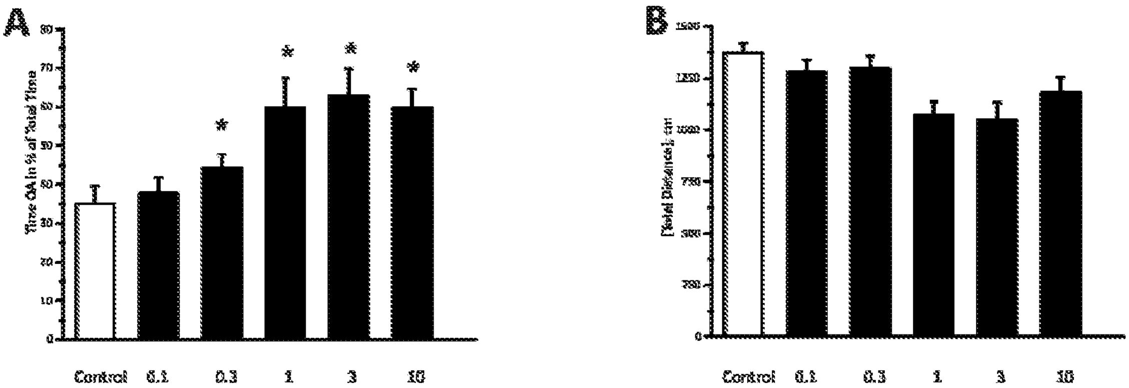


Figure 10:

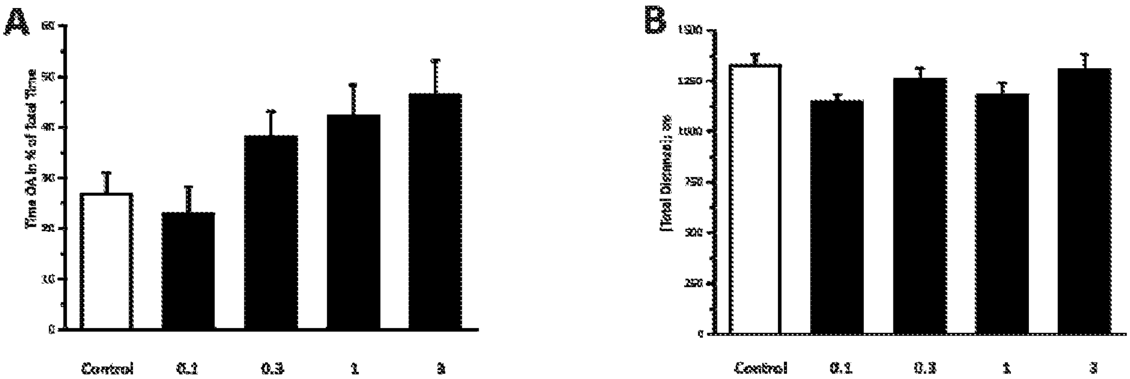


Figure 11:

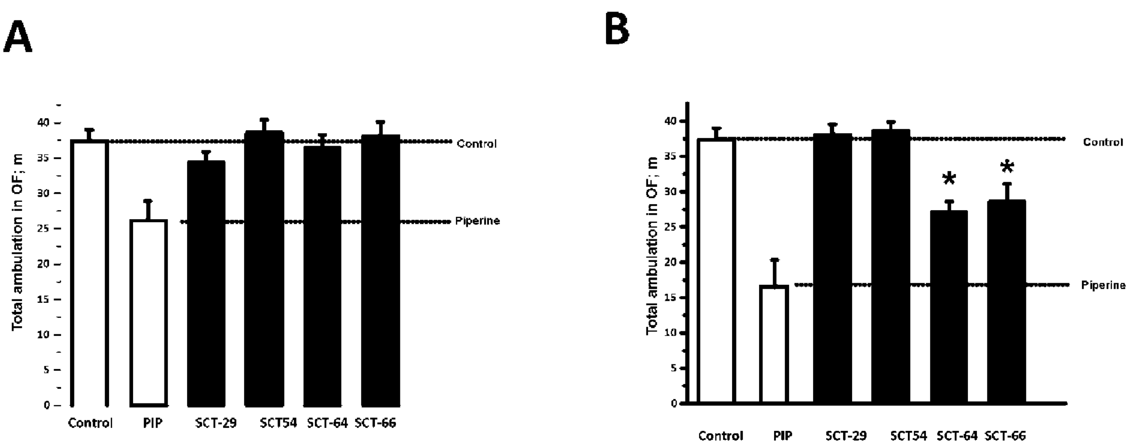

Figure 12

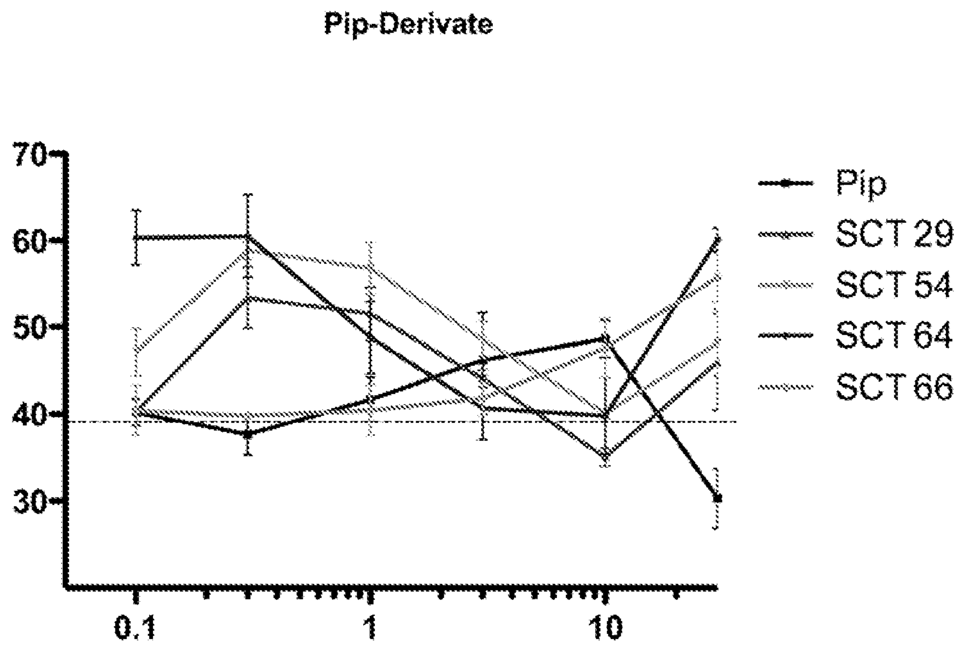

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JOHNSTON, G. A. R. ; HANRAHAN, J. R. ; CHEBIB, M. ; DUKE, R. K. ; MEWETT, K. N.** *Adv. Pharmacol.,* 2006, vol. 54, 286-316
- **TSANG, S. Y. ; ; XUE, H.** *Curr. Pharm. Design,* 2004, vol. 10, 1035-1044
- **PEDERSEN, M. E. ; METZLER, B. ; STAFFORD, G. I. ; VAN STADEN, J. ; JAEGER, A. K. ; RASMUSSEN, H. B.** *Molecules,* 2009, vol. 14, 3833-3843
- **PEI, Y. Q.** *Epilepsia,* 1983, vol. 24, 177-182
- **SZALLASI, A.** *Trends in Pharmacological Sciences,* 2005, vol. 26, 437-439
- **SUNILA, E. ; KUTTAN, G. J.** *Ethnopharmacol,* 2004, vol. 90, 339-346
- **WHITING, P. J.** *Curr. Opin. Pharmacol.,* 2006, vol. 6, 24-29
- **KAPLAN, E. M. ; DUPONT, R. L.** *Curr. Med. Res. Opin.,* 2005, vol. 21, 941-950
- **RUPPRECHT, R. ; ESER, D. ; ZWANZGER, P. ; MOELLER, H.-J.** *World J. Biol. Psychiatry,* 2006, vol. 7, 231-237
- **MCNAMARA, F. N. ; RANDALL, A. ; GUNTHORPE, M. J.** *Brit. J. Pharmacol.,* 2005, vol. 144, 781-790
- **ANAND, P. ; KUNNUMAKKARA, A. B. ; NEWMAN, R. A. ; AGGARWAL, B. B.** *Mol. Pharm.,* 2007, vol. 4, 807-818
- **BABURIN, I. ; BEYL, S. ; HERING, S.** *Pflug. Arch. Eur. J. Phy.,* 2006, vol. 453, 117-123
- **KHOM, S. ; BABURIN, I. ; TIMIN, E. N. ; HOHAUS, A. ; SIEGHART, W. ; HERING, S.** *Mol. Pharmacol.,* 2006, vol. 69, 640-649
- **KHOM S. ; BABURIN I. ; TIMIN E. ; HOHAUS A. ; TRAUNER G. ; KOPP B. ; HERING S.** *Neuropharmacology,* 2007, vol. 53 (1), 178-87
- **HU HZ ; GU Q ; WANG C ; COLTON CK ; TANG J ; KINOSHITA-KAWADA M ; LEE LY ; WOOD JD ; ZHU MX.** *J Biol Chem.,* 20 August 2004, vol. 279 (34), 35741-8
- **ZAUGG J. ; BABURIN I. ; STROMMER B. ; KIM H.-J ; HERING S. ; HAMBURGER M.** *J Nat Prod.,* 2010, vol. 73 (2), 185-91
- **KHOM S ; STROMMER B ; RAMHARTER J ; SCHWARZ T ; SCHWARZER C ; ERKER T ; ECKER GF ; MULZER J ; HERING S.** *Br J Pharmacol.,* September 2010, vol. 161 (1), 65-78
- **LOACKER S ; SAYYAH M ; WITTMANN W ; HERZOG H ; SCHWARZER C.** *Brain,* 08 March 2007, vol. 130, 1017-28